# EUROPEAN PATENT APPLICATION

(11) **EP 4 442 198 A1**
(43) Date of publication of application: **09.10.2024**
(21) Application number: 22899757.3
(22) Date of filing: 18.03.2022
(51) Int. Cl.: A61B 5/145

(54) **IMPLANTATION DEVICE, NEEDLE, SENSOR BASE, EMITTER ASSEMBLY, STERILIZATION BOX, AND IMPLANTATION SYSTEM**

(30) Priority: 30.11.2021 CN 202111437974; 30.11.2021 CN 202210190806; 30.11.2021 CN 202210187975; 30.11.2021 CN 202210190799
(71) Applicant: Shanghai Microport Lifesciences Co., Ltd., Shanghai 201318 (CN)
(72) Inventor: FU, Xiangyang, Shanghai 201318 (CN); CHEN, Xun, Shanghai 201318 (CN); HAO, Xuan, Shanghai 201318 (CN); LIU, Wenjie, Shanghai 201318 (CN); QIU, Dan, Shanghai 201318 (CN)
(74) Representative: Patentship Patentanwaltsgesellschaft mbH
(86) International application number: PCT/CN2022/081738
(87) International publication number: WO 2023/097934

(57) **Abstract**

The present application provides an implantation device, a needle, a sensor base, an emitter assembly, a sterilization box and an implantation system. The implantation device includes a base body, a press member, an ejector assembly and a first potential energy storage element. The base body has axially opposing first and second ends and includes a first stopper. The ejector assembly is disposed so as to be movable along the axis of the base body. Moreover, the ejector assembly is configured to be connected to the needle assembly and detachably connected to the sensor base assembly. The first potential energy storage element stores potential energy as the ejector assembly moves toward the first end. Once the ejector assembly moves toward the first end and exceeds a first predetermined position, it is restricted by the first stopper from moving toward the second end so as not exceed the first predetermined position. When the press member is pressed, the ejector assembly is released from the first stopper, and the first potential energy storage element releases potential energy, causing the ejector assembly, along with the needle assembly and the sensor base assembly, to move toward the second end.

## Description

### TECHNICAL FIELD

The present application relates to the field of medical instruments and, in particular, to an implantation device, a needle, a sensor base, an emitter assembly, a sterilization box, and an implantation system.

### BACKGROUND

Increasing improvement of our living conditions and abundance of our daily diet have brought about an increase in the incidence of diabetes, leading to a more and more urgent demand for continuous glucose monitoring (CGM). At present, most products for this purpose are disposable implants for single use. These are expensive and their use is considerably wasteful and environmentally unfriendly.

### SUMMARY OF THE INVENTION

It is an object of the present application to provide an implantation device, a needle, a sensor base, an emitter assembly, a sterilization box and an implantation system, which overcome the aforementioned problems of high cost and environmental unfriendliness with conventional single-use implants.

To this end, in a first aspect of the present application, there is provided an implantation device comprising a base body, a press member, an ejector assembly and a first potential energy storage element,
the base body having a first end and a second end, which oppose each other along an axis of the base body, the base body comprising a first stopper, the ejector assembly disposed so as to be movable along the axis of the base body, the ejector assembly configured to be connected to a needle assembly and detachably connected to a sensor base assembly,
the first potential energy storage element configured to store potential energy as the ejector assembly moves toward the first end, the ejector assembly configured to be restricted by the first stopper from moving toward the second end once the ejector assembly moves toward the first end and not exceed a first predetermined position,
when the press member is pressed down, the ejector assembly is released from the first stopper, and the first potential energy storage element releases potential energy to cause the ejector assembly to move toward the second end, along with the needle assembly and the sensor base assembly.

Optionally, the ejector assembly comprises a second stopper and a second potential energy storage element,
the second stopper disposed so as to be movable along a radial direction of the base body,
the second potential energy storage element configured to store potential energy as the ejector assembly moves in a direction from the second end to the first end and release potential energy, when the ejector assembly moves toward the first end and exceeds the first predetermined position, to cause the second stopper to move with respect to the base body along the radial direction of the base body to a second predetermined position, the second stopper, when at the second predetermined position, configured to abut against the first stopper to restrict the ejector assembly from moving toward the second end.

Optionally, with the ejector assembly being located at the first predetermined position and the second stopper at the second predetermined position, when the press member is pressed, the second stopper is caused to move along the radial direction of the base body away from the second predetermined position so that the second stopper does not abut against the first stopper.

Optionally, the base body comprises a first sloped surface which is sloped inwardly as extending toward the second end, and/or the second stopper comprises a second sloped surface which is sloped outwardly as it extending toward the first end,
wherein during movement of the ejector assembly toward the first end, the second stopper is restricted by the first sloped surface and/or the second sloped surface to gradually move inwardly along the radial direction of the base body so that the second potential energy storage element to store potential energy.

Optionally, the ejector assembly is configured to be movably connected to the needle assembly and comprise a third stopper configured to restrict the needle assembly from moving toward the second end so as not exceed a third predetermined position relative to the ejector assembly.

Optionally, the third stopper is disposed so as to be movable radially with respect to the base body, wherein the ejector assembly comprises a third potential energy storage element configured to store potential energy as the needle assembly moves toward the first end relative to the ejector assembly,
when the needle assembly moves toward the first end and exceeds the third predetermined position relative to the ejector assembly, the third potential energy storage element releases potential energy to cause the third stopper to move along a radial direction of the base body to a fourth predetermined position, and wherein the third stopper, when at the fourth predetermined position, is configured to abut against the needle assembly to restrict the needle assembly from moving toward the second end so as not exceed the third predetermined position relative to the ejector assembly.

Optionally, the third stopper comprises a third sloped surface which is sloped inwardly as extending toward the second end,
wherein as the needle assembly moves toward the first end relative to the ejector assembly, with the third sloped surface abutting against the needle assembly, the third stopper moves along the radial direction of the base body to cause the third potential energy storage element to store potential energy.

Optionally, the ejector assembly is detachably connected to the needle assembly, wherein the third stopper is configured to release the needle assembly when abutting against a needle recovery member so that the needle assembly is separated from the ejector assembly.

Optionally, the base body comprises a fourth stopper configured to restrict the ejector assembly from moving toward the second end so as not exceed a fifth predetermined position.

Optionally, the ejector assembly comprises a fifth stopper,
when the ejector assembly is located at the first predetermined position, the fifth stopper restricts the sensor base assembly from moving toward the second end relative to the ejector assembly,
the fifth stopper less restricts the sensor base assembly as the ejector assembly moves from the first predetermined position toward the second end.

Optionally, the base body comprises a fourth sloped surface which is sloped inwardly as extending toward the second end,
wherein the fifth stopper is disposed so as to movable along a radial direction of the base body;
as the ejector assembly moves toward the first end, the fifth stopper is restricted by the fourth sloped surface to gradually move inwardly along the radial direction of the base body into snap-engagement with the sensor base assembly so that the sensor base assembly is restricted from moving toward the second end relative to the ejector assembly; and
as the ejector assembly moves toward the second end, the fifth stopper gradually moves outwardly along the radial direction of the base body so that pressure of snap-engagement between the fifth stopper and the sensor base assembly is lowered for releasing the sensor base assembly from the ejector assembly.

Optionally, the fifth stopper comprises a fifth sloped surface which is sloped outwardly as extending toward the first end and configured to match with, and abut against, the fourth sloped surface.

Optionally, the ejector assembly comprises a fourth potential energy storage element configured to provide potential energy to the fifth stopper as the ejector assembly moves toward the second end so that, the fifth stopper is caused to gradually move outwardly along the radial direction of the base body.

Optionally, the ejector assembly further comprises a fifth potential energy storage element,
when the ejector assembly is assembled with the sensor base assembly, the fifth potential energy storage element stores potential energy,
when the ejector assembly is separated from the sensor base assembly, the fifth potential energy storage element releases potential energy to cause the sensor base assembly to move away from the ejector assembly.

To the above end, in a second aspect of the present application, there is provided a needle assembly for use with the implantation device as defined above. The needle assembly comprises a shell and a needle body.

The shell is connected to the needle body.

The shell is configured to be connected to the ejector assembly of the implantation device. The shell is also configured to be detachably connected to the sensor base assembly.

Optionally, the needle assembly further comprises a needle sheath and a sixth potential energy storage element, the sixth potential energy storage element fixedly connected at opposite ends thereof to the needle sheath and the needle body respectively,
when the shell is assembled with the sensor base assembly, the sixth potential energy storage element stores potential energy so that the needle body protrudes out of the needle sheath along an axis of the shell,
when the shell is separated from the sensor base assembly, the sixth potential energy storage element releases potential energy so that the needle body is gradually received into the needle sheath along the axis of the shell.

Optionally, the needle body is connected to the shell so as to be movable along the axis thereof, and the needle sheath is fixedly connected to the shell,
when the shell is assembled with the sensor base assembly, the sixth potential energy storage element stores potential energy so that the needle body to protrude out of the needle sheath along the axis of the shell, and
when the shell is separated from the sensor base assembly, the sixth potential energy storage element releases potential energy so that the needle body is received into the needle sheath.

Optionally, the needle body comprises a needle holder, wherein one end of the sixth potential energy storage element is connected to the needle holder, and a further end of the sixth potential energy storage element is connected to the needle sheath.

Optionally, the needle body is fixedly connected to the shell, and the needle sheath being is connected to the shell so as to be movable along the axis of the shell,
when the shell is assembled with the sensor base assembly, the sixth potential energy storage element stores potential energy so that the needle body protrudes out of the needle sheath as the needle sheath is restricted in position by the shell, and
wherein when the shell is separated from the sensor base assembly, the needle sheath is released from the shell, and the sixth potential energy storage element releases potential energy so that the needle sheath is caused to move along the axis of the shell over the needle body.

Optionally, the shell comprises a sixth stopper,
when the shell is assembled with the sensor base assembly, the sixth stopper is configured to abut against the needle body or the needle sheath to cause the sixth potential energy storage element to store potential energy so that the needle body or the needle sheath is restricted in axial position relative to the shell,
when the shell is separated from the sensor base assembly, the sixth stopper moves away from abutment against the needle body or the needle sheath to cause the sixth potential energy storage element to release potential energy so that the needle body or the needle sheath is released from positional restriction.

Optionally, the shell comprises a seventh potential energy storage element and a first snap engagement member, the first snap engagement member configured to be detachable connected with the sensor base assembly along the axis of the shell, when the first snap engagement member is assembled with the sensor base assembly, the seventh potential energy storage element stores potential energy and provides potential energy to the first snap engagement member so that the first snap engagement member is caused to move along a radial direction of the shell to abut against the sensor base assembly.

Optionally, the shell comprises a sixth stopper, wherein when the first snap engagement member is separated from the sensor base assembly, the seventh potential energy storage element releases potential energy to cause the sixth stopper to move so that the needle body or the needle sheath is released from positional restriction.

Optionally, the shell is detachably connected to the ejector assembly along an axis thereof, wherein the needle assembly further comprises an eighth potential energy storage element,
when the shell is assembled with the ejector assembly, the eighth potential energy storage element stores potential energy,
when the shell is separated from the ejector assembly, the eighth potential energy storage element releases potential energy to cause the shell to move along the axis of the ejector assembly so that the shell is separated from the ejector assembly.

Optionally, the shell comprises a seventh stopper configured to abut against the third stopper of the ejector assembly so that the shell is restricted from moving toward the second end and does not exceed the third predetermined position relative to the ejector assembly.

Optionally, the needle assembly is detachably connected to the ejector assembly, when the seventh stopper moves away from abutment against the third stopper, the shell is no longer restricted from movement, so that the needle assembly is separated from the ejector assembly.

To the above end, in a third aspect of the present application, there is provided a sensor base assembly for use with the implantation device as defined above and the needle assembly as defined above. The sensor base assembly comprises a base and an adhesive layer.

The base has a third end and a fourth end, which oppose each other along an axis of the base. The base is configured to be detachably connected, at a side where the third end is located, to the needle assembly.

The adhesive layer is provided at a side of the base where the fourth end is located.

When the ejector assembly is located at the first predetermined position, the base is configured for snap engagement with the fifth stopper to restrict the sensor base assembly from moving toward the second end relative to the ejector assembly,
wherein as the ejector assembly moves from the first predetermined position toward the second end, the base is configured to disengage from the fifth stopper to release the sensor base assembly from the fifth stopper.

Optionally, the base comprises a second snap engagement member configured to be detachable connected with the first snap engagement of the needle assembly along an axis of the shell of the needle assembly.

Optionally, the base is further configured to detachably connected, at the third end, to an emitter assembly, wherein the base comprises a third snap engagement member configured for snap engagement with the emitter assembly.

Optionally, the sensor base assembly further comprises a flexible conductor and a sensor component, the sensor component configured to be implanted together with the needle body in the needle assembly to a predetermined site so that the flexible conductor is electrically connected to the sensor component and to be detachably connected to the emitter assembly.

To the above end, in a fourth aspect of the present application, there is provided an emitter assembly for use with the sensor base assembly as defined above. The emitter assembly comprises a fourth snap engagement member configured for snap engagement with the third snap engagement member of the sensor base assembly.

To the above end, in a fifth aspect of the present application, there is provided a sterilization box assembly for accommodating the needle assembly as defined above and the sensor base assembly as defined above.

Optionally, the sterilization box assembly comprises a needle recovery member configured to be brought into abutment against the third stopper in the implantation device to release the needle assembly from the third stopper to allow the needle assembly is separated from the implantation device.

Optionally, the sterilization box assembly is further be configured to accommodate the needle assembly that has been separated from the implantation device.

Optionally, the sterilization box assembly comprises a sterilization box shell comprising a first cavity and a second cavity, the first cavity configured for accommodating therein the needle assembly and the sensor base assembly, which are assembled together, wherein the needle recovery member is disposed at the side of the sterilization box shell opposite to the first cavity, and after the needle recovery member is brought into abutment against the third stopper of the implantation device and thereby releases the needle assembly is released from the third stopper, the needle assembly moves from the implantation device into the second cavity.

Optionally, the sterilization box assembly further comprises a foil seal configured to be sealingly connected to the sterilization box shell to close the first cavity.

Optionally, the sterilization box assembly further comprises a top protective cover provided on an inner surface of the foil seal, the top protective cover configured to restrict movement of the needle assembly and the sensor base assembly.

Optionally, the sterilization box assembly further comprises a retaining receptacle arranged at the bottom of the sterilization box assembly in alignment with the needle assembly when the latter is falling.

To the above end, in a sixth aspect of the present application, there is provided an implantation system comprising the implantation device as defined above, the needle assembly as defined above, the sensor base assembly as defined above and the sterilization box assembly as defined above.

The sterilization box assembly is configured to accommodate the needle assembly and the sensor base assembly, which are assembled together.

The implantation device is configured to be inserted along the direction from the second end to the first end, into the needle assembly and the sensor base assembly that are accommodated in the sterilization box assembly.

The needle assembly is configured to be separated from the sensor base assembly after the sensor base assembly is attached to a predetermined implantation site.

The emitter assembly is configured to be assembled with the sensor base assembly after the needle assembly is separated from the sensor base assembly.

In summary, the present application provides an implantation device, a needle, a sensor base, an emitter assembly, a sterilization box and an implantation system. The implantation device includes a base body, a press member, an ejector assembly and a first potential energy storage element. The base body has axially opposing first and second ends and includes a first stopper. The ejector assembly is disposed so as to be movable along the axis of the base body. Moreover, the ejector assembly is configured to be connected to the needle assembly and detachably connected to the sensor base assembly. The first potential energy storage element stores potential energy as the ejector assembly moves toward the first end. Once the ejector assembly moves toward the first end and exceeds a first predetermined position, it is restricted by the first stopper from moving toward the second end. When the press member is pressed, the ejector assembly is released from the first stopper, and the first potential energy storage element releases potential energy that it has stored, causing the ejector assembly, along with the needle assembly and the sensor base assembly, to move toward the second end.

With this arrangement, when the press member is pressed down, the first potential energy storage element releases potential energy that it has stored, causing the ejector assembly to move, along with the needle assembly and the sensor base assembly, to complete the implantation. Moreover, since the ejector assembly is detachable from the sensor base assembly, after the implantation is completed, the implantation device and the needle assembly can be both removed. What is to be implanted is only the sensor base assembly, and after the needle assembly is removed, the implantation device can be reused many times, effectively reducing the cost and the influence on the environment.

### BRIEF DESCRIPTION OF THE DRAWINGS

Those of ordinary skill in the art would appreciate that the following drawings are presented to enable a better understanding of the present application and do not limit the scope thereof in any sense, in which:
Fig. 1 schematically illustrates an implantation system according to an embodiment of the present application;
Fig. 2 schematically illustrates an assembled sterilization box according to an embodiment of the present application;
Fig. 3 is a schematic view of an axial cross-section of an implantation device according to an embodiment of the present application;
Fig. 4 is a schematic view of another axial cross-section of the implantation device, which is oriented in a different direction, according to an embodiment of the present application;
Fig. 5 is an enlarged partial view of Fig. 3;
Fig. 6 is a top view of an implantation device according to an embodiment of the present application;
Fig. 7 is a schematic view of an axial cross-section of an assembly of an implantation device, a needle assembly and a sensor base assembly, in which an ejector assembly is located at a first predetermined position, according to an embodiment of the present application;
Fig. 8 is a schematic view of another axial cross-section of the assembly of Fig. 7, which is oriented in a different direction;
Fig. 9 is a schematic view of an axial cross-section of an assembly of an implantation device, a needle assembly and a sensor base assembly, in which an ejector assembly is located at a fifth predetermined position, according to an embodiment of the present application;
Fig. 10 is a schematic view of another axial cross-section of the assembly of Fig. 9, which is oriented in a different direction;
Fig. 11 is a schematic view of an axial cross-section of an assembly of a needle assembly and a sensor base assembly according to an embodiment of the present application;
Fig. 12 is a schematic view of another axial cross-section of the assembly of Fig. 11, which is oriented in a different direction;
Fig. 13 is a schematic axial cross-sectional view of the needle assembly and the sensor base assembly of Fig. 12, which have been separated from each other;
Fig. 14 is a schematic view of an axial cross-section of an implantation device, a needle assembly and a sensor base assembly which has been separated from the implantation device and the needle assembly, according to an embodiment of the present application;
Fig. 15 is a schematic view of another axial cross-section of the implantation device, the needle assembly and the sensor base assembly of Fig. 14, which is oriented in a different direction;
Fig. 16 is an enlarged partial view of Fig. 14;
Fig. 17 is a schematic axial cross-sectional view of an emitter assembly according to an embodiment of the present application;
Fig. 18 is a top view of an emitter assembly according to an embodiment of the present application;
Fig. 19 schematically illustrates an emitter assembly and a sensor base assembly, which have not been assembled together yet, according to an embodiment of the present application;
Fig. 20 is a schematic illustration of the emitter assembly and the sensor base assembly of Fig. 19, which have been assembled together;
Fig. 21 is a schematic axial cross-sectional view of Fig. 20;
Fig. 22 is another schematic illustration of the emitter assembly and the sensor base assembly, which have not been assembled together yet, as viewed in another direction than that of Fig. 19, according to an embodiment of the present application;
Fig. 23 is a schematic illustration of the emitter assembly and the sensor base assembly of Fig. 22, which have been assembled together;
Fig. 24 is a schematic axial cross-sectional view of Fig. 23;
Fig. 25 schematically illustrates an emitter assembly sealingly assembled with a sensor base assembly according to an embodiment of the present application;
Fig. 26 is a schematic transverse cross-sectional view of Fig. 25;
Fig. 27 is another schematic transverse cross-sectional view of Fig. 25;
Fig. 28 schematically illustrates an implantation device and a sterilization box assembly, which have not been assembled together yet, according to an embodiment of the present application;
Fig. 29 is an enlarged partial view of the implantation device of Fig. 28;
Fig. 30 schematically illustrates an implantation device and a sterilization box assembly assembled with the implantation device according to an embodiment of the present application;
Fig. 31 is an enlarged partial view of the implantation device of Fig. 30; and
Fig. 32 schematically illustrates an implantation device and a needle assembly, which have been separated from each other, according to an embodiment of the present application.

### List of Reference Numerals

100 - implantation device; 101 - base body; 101a - first end; 101b - second end; 1011 - fourth stopper; 1012 - slide channel; 1013 - first sloped surface; 1014 - first stopper; 1015 - elastic leg; 1016 - fourth sloped surface; 102 - press member; 111 - ejector assembly; 1111 - slider; 112 - first potential energy storage element; 121 - fifth stopper; 1211 - fifth sloped surface; 122 - fourth potential energy storage element; 131 - second stopper; 1311 - second sloped surface; 132 - second potential energy storage element; 141 - third stopper; 1411 - third sloped surface; 1412 - stop ledge; 1413 - stop surface; 142 - third potential energy storage element; 143 - fifth potential energy storage element;
200 - assembled sterilization box; 210 - sterilization box assembly; 211 - sterilization box shell; 2111 - needle recovery member; 2112 - retaining receptacle; 212 - foil seal; 213 - top protective cover;
220 - sensor base assembly; 221 - sensor component; 222 - base; 222a - third end; 222b - fourth end; 2221 - third snap engagement member; 2222 - second snap engagement member; 223 - adhesive layer;
230 - needle assembly; 240 - elastic seal; 242 - flexible conductor; 251 - shell; 2512 - seventh stopper; 2513 - sixth sloped surface; 252 - seventh potential energy storage element; 2521 - first snap engagement member; 2522 - eighth potential energy storage element; 2523 - sixth stopper; 253 - needle sheath; 261 - needle holder; 262 - sixth potential energy storage element; 263 - needle body; 300 - emitter assembly; 301 - top housing; 302 - bottom housing; 3021 - fourth snap engagement member; 310 - circuit board; 320 - battery.

### DETAILED DESCRIPTION

Objects, features and advantages of the present application will become more apparent upon reading the following description with reference to the accompanying drawings, which illustrate particular embodiments thereof. Note that the figures are provided in a very simplified form not necessarily drawn to exact scale for the only purpose of helping to explain the disclosed embodiments in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents. As used herein, the term "or" is generally employed in the sense of "and/or", "some" of "at least one" and "at least two" of "two or more". Additionally, the use of the terms "first", "second" and "third" herein is intended for illustration only and is not to be construed as denoting or implying relative importance or as implicitly indicating the numerical number of the referenced item. Accordingly, defining an item with "first" , "second" or "third" is an explicit or implicit indication of the presence of one or at least two such items. The term "proximal end" usually refers to an end closer to an operator, and the term "distal end" usually refers to an end closer to a lesion in a patient. The terms "one end" and "the other end", as well as "proximal end" and "distal end", are used to generally refer to opposing ends including the opposing endpoints, rather than only to the endpoints. The terms "mount", "couple", "connect" and any variants thereof should be interpreted in a broad sense. For instance, a connection may be a permanent, detachable or integral connection, or a mechanical or electrical connection, or a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location relative to the other element, unless the context clearly dictates otherwise. Those of ordinary skill in the art can understand the specific meanings of the above-mentioned terms herein, depending on their context.

The present application seeks to provide an implantation device, a needle assembly, a sensor base assembly, an emitter assembly, a sterilization box and an implantation system, which overcome the aforementioned problems of high cost and environmental unfriendliness with conventional single-use implants.

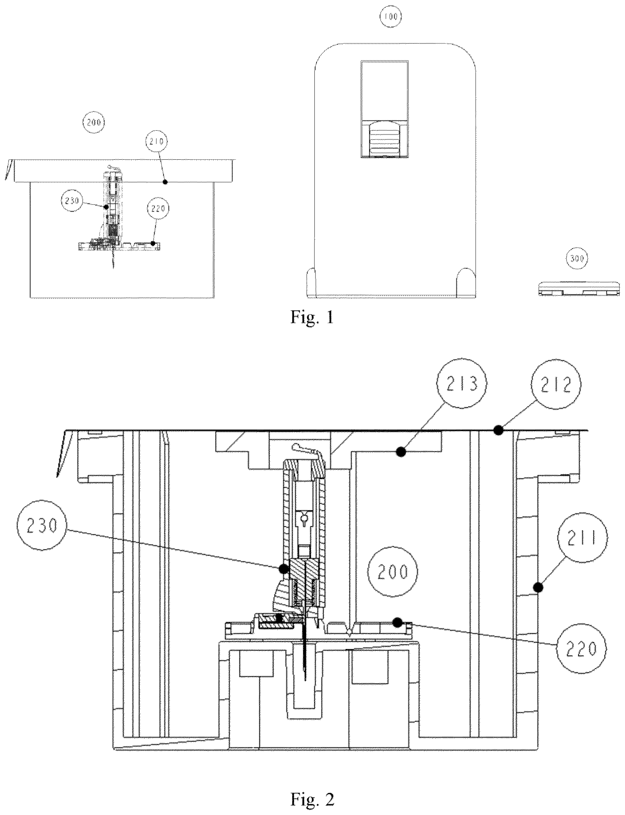

As shown in Fig. 1, embodiments of the present application provide an implantation system comprising an implantation device 100, a sterilization box assembly 210, an emitter assembly 300, a needle assembly 230 and a sensor base assembly 220. In an initial configuration, the needle assembly 230 and the sensor base assembly 220 can be received in the sterilization box assembly 210, forming an assembled sterilization box 200. During use, the implantation device 100 is inserted into the sterilization box assembly 210 so that the needle assembly 230 and the sensor base assembly 220 are loaded into the implantation device 100. The implantation device 100 is then utilized to implant the needle assembly 230 and the sensor base assembly 220 to a predetermined implantation site of a target object. The implantation device 100 and the needle assembly 230 are then withdrawn, with the sensor base assembly 220 remaining at the predetermined implantation site. In order to complete the implantation process, the emitter assembly 300 is then assembled onto the sensor base assembly 220.

Steps of using the implantation system according to an embodiment of the present application will be exemplified with reference to the annexed figures. The steps of using the implantation system mainly include capture, release, separation, assembly and recovery steps. It should be noted that components involved in these steps will be described and illustrated in greater detail after the description of the steps.

### <Capture Step>

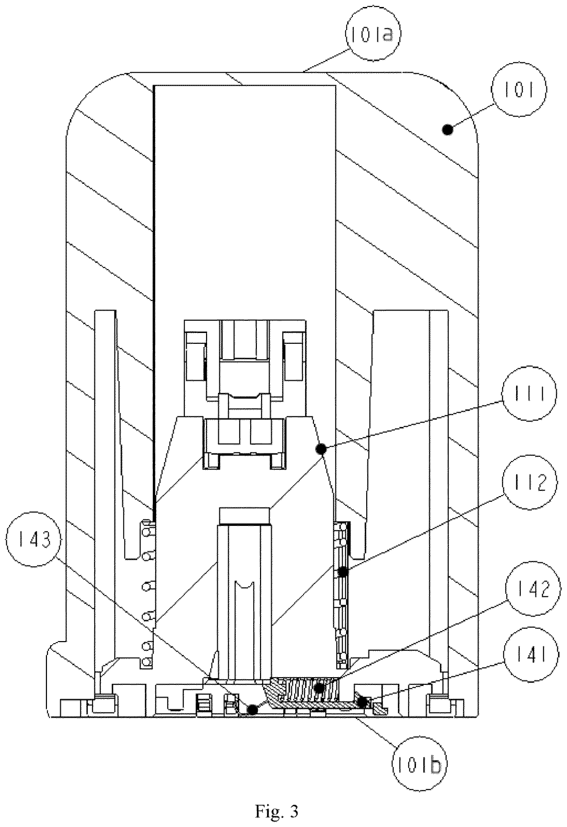

Reference is made to Figs. 2 and 3. Fig. 2 shows the sterilization box 200 in an initial configuration, and Fig. 3 shows the implantation device 100 in an initial configuration.

After a foil seal 212 and a top protective cover 213 are torn off, the needle assembly 230 and the sensor base assembly 220 are exposed.

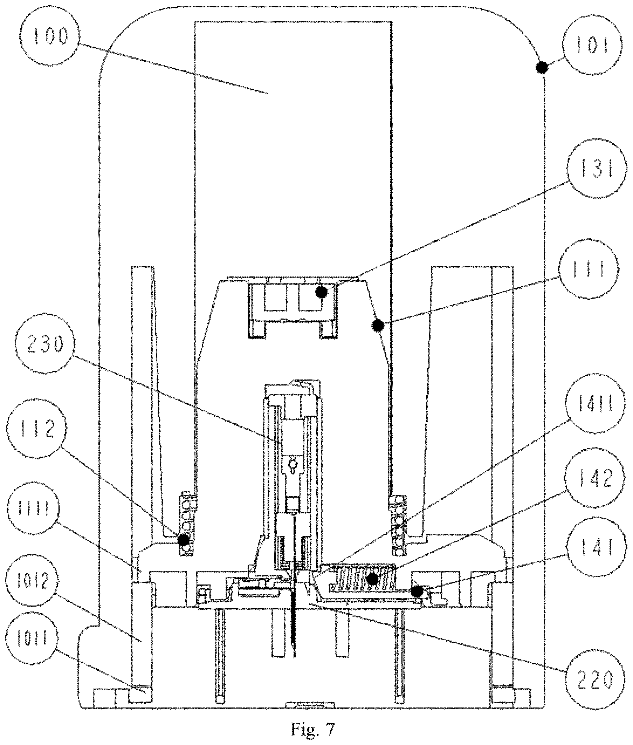

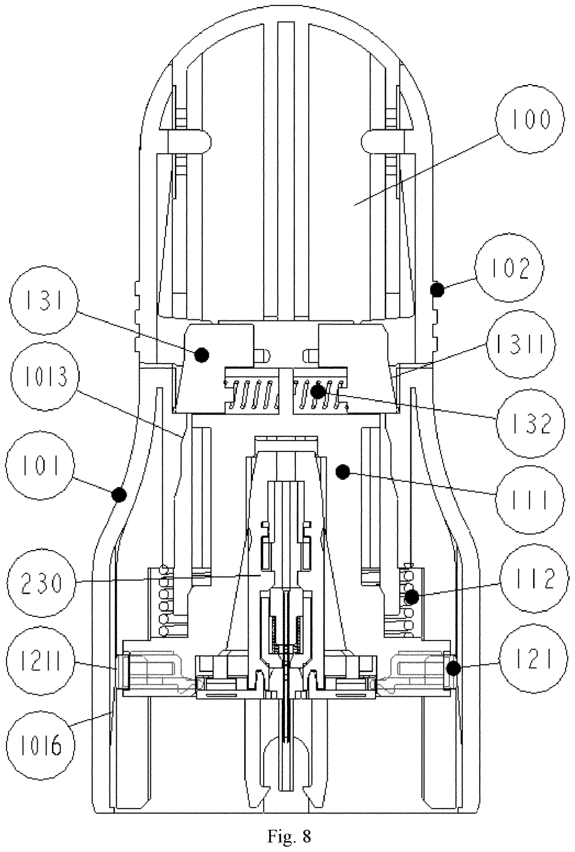

The implantation device 100 is vertically oriented with its second end 101b facing toward the sterilization box 200 (i.e., as shown in Fig. 3) and moved in a direction from its second end 101b to its first end 101a toward the bottom of the sterilization box 200 (as viewed in the orientation of Fig. 2) so that a portion of the implantation device 100 around the second end 101b is inserted into the sterilization box 200. As a result, the needle assembly 230 is inserted into an ejector assembly 111. After that, the implantation device 100 is further pushed toward the bottom of the sterilization box 200, causing the ejector assembly 111 to move under the action of a reaction force from a stop ledge within a shell 211 of the sterilization box toward a first end 101a of a base body 101 (i.e., the ejector assembly 111 is pushed upwards) to a first predetermined position. Upon this happening, a second potential energy storage element 132 releases potential energy that it has stored, resulting in the ejector assembly 111 being snap retained by a first stopper 1014, as shown in Figs. 7 and 8. In this process, a first potential energy storage element 112 stores potential energy.

After the needle assembly 230 is inserted into the ejector assembly 111, as a result of the implantation device 100 being pushed toward the bottom of the sterilization box 200, the needle assembly 230 moves toward the first end 101a until it reaches a third predetermined position, where it is snap retained by a third stopper 141 (more precisely, by a seventh stopper 2512 and a stop ledge 1412), as shown in Fig. 7. In this process, a third potential energy storage element 142 stores potential energy. It should be noted that the needle assembly 230 may reach the third predetermined position before the ejector assembly 111 starts moving toward the first end 101a of the base body 101, or after the ejector assembly 111 reaches the first predetermined position, or when the ejector assembly 111 is located between said two positions.

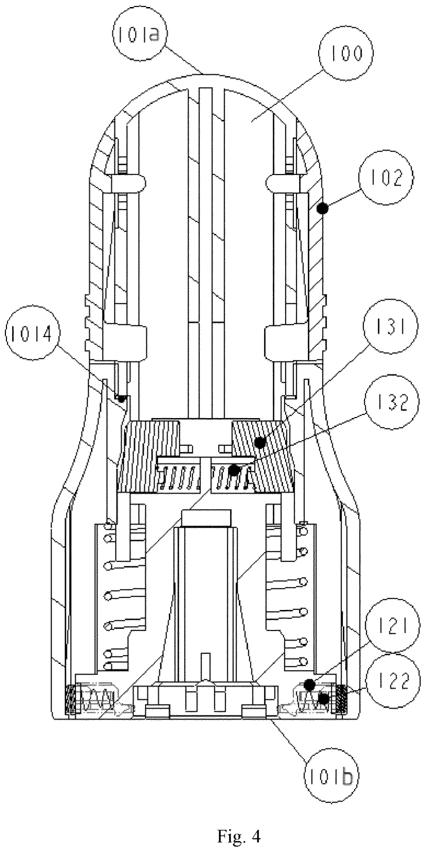

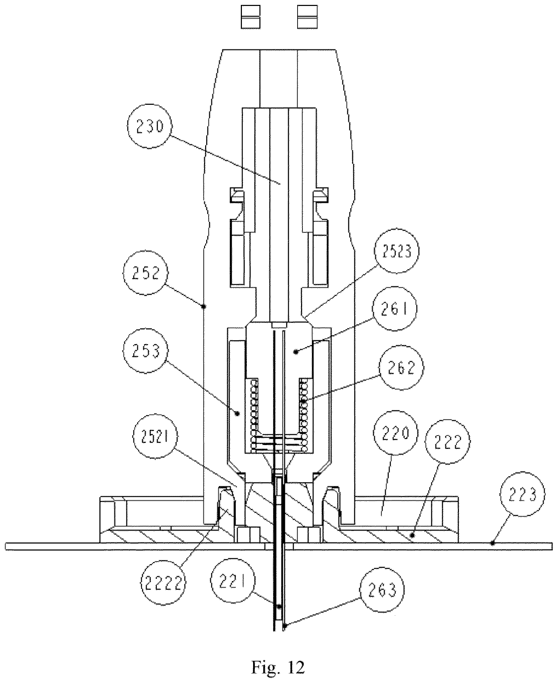

The sensor base assembly 220 is initially assembled with the needle assembly 230, with first snap engagement members 2521 being snap-engaged with second snap engagement members 2222, as shown in Fig. 12. Further, referring to Figs. 4 and 8, as the ejector assembly 111 moves toward the first end 101a of the base body 101, a fifth stopper 121 is guided by a fourth sloped surface 1016 and/or a fifth sloped surface 1211 to gradually move inwardly. In this process, a fourth potential energy storage element 122 is pushed to store potential energy. In this way, when the ejector assembly 111 is located at the first predetermined position, the sensor base assembly 220 is additionally retained, ensuring that the sensor base assembly 220 will not dislodge from the implantation device 100 after the needle assembly 230 and the sensor base assembly 220 are loaded into the implantation device 100.

Finally, as shown in Figs. 7 and 8, the sensor base assembly 220 and the needle assembly 230 are captured by the implantation device 100.

### <Release Step>

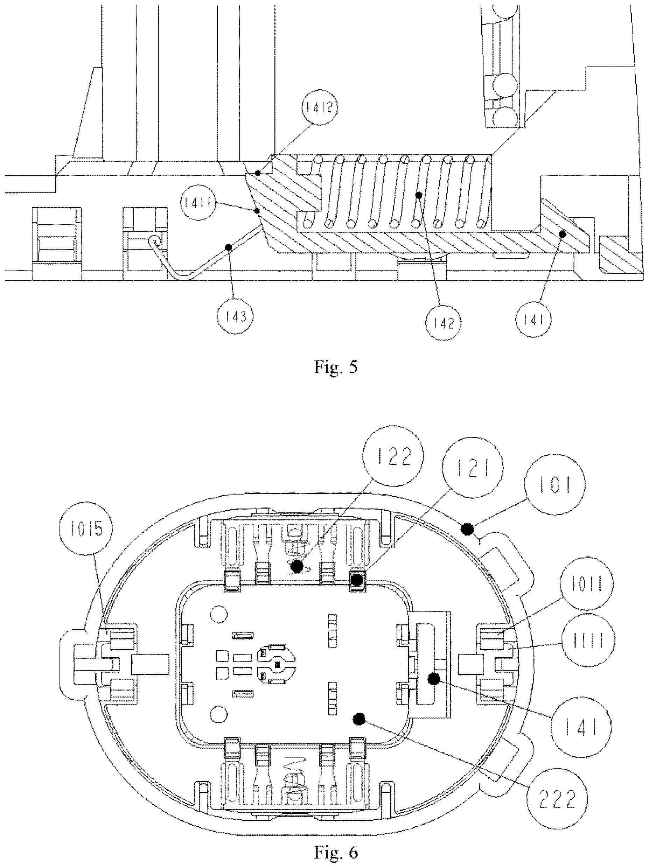

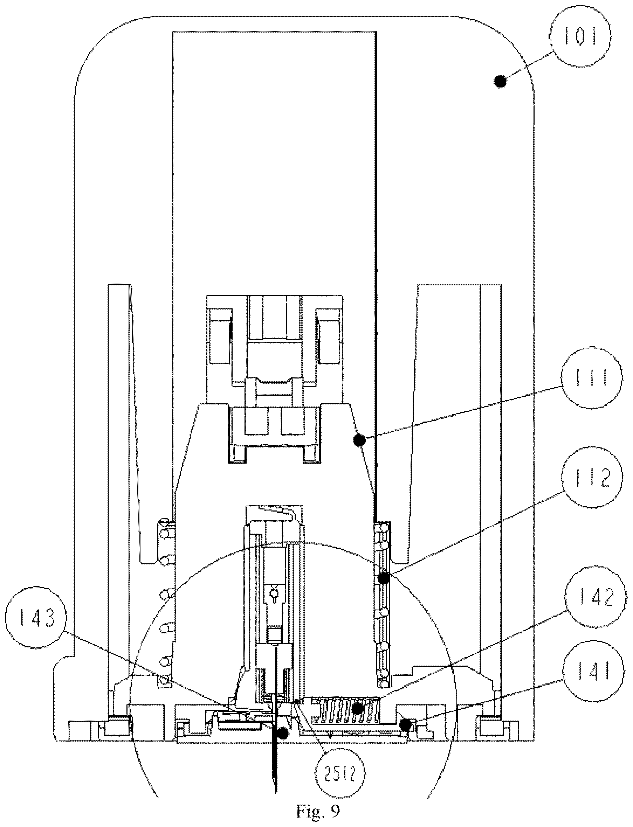

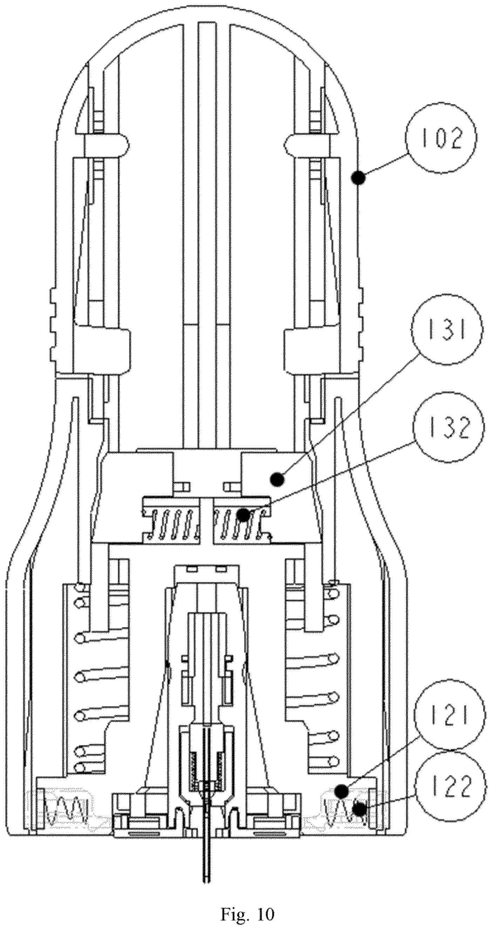

The assembly shown in Figs. 7 and 8 is moved to the predetermined implantation site, and a press member 102 is pressed down to push the second stopper 131 inwardly to release the ejector assembly 111 from the first stopper 1014. In response, the first potential energy storage element 112 releases potential energy that it has stored, ejecting the ejector assembly 111 toward the second end 101b. When the ejector assembly 111 reaches a fifth predetermined position, it is retained by fourth stoppers 1011 (see Figs. 6 and 7). At this point, the implantation device 100 generally appears as shown in Figs. 9 and 10. Further, in this process, the fourth potential energy storage element 122 releases potential energy that it has stored, urging the fifth stopper 121 outwardly. Consequently, it no longer additionally retains the sensor base assembly 220.

The needle assembly 230 remains inserted in the ejector assembly 111 and is thereby ejected along with the ejector assembly 111, causing a sensor component 221 of the sensor base assembly 220 to pierce the skin at the predetermined implantation site.

The sensor base assembly 220 remains assembled with the needle assembly 230. Accordingly, an adhesive layer 223 adheres to the skin surface at the predetermined implantation site.

### <Separation Step>

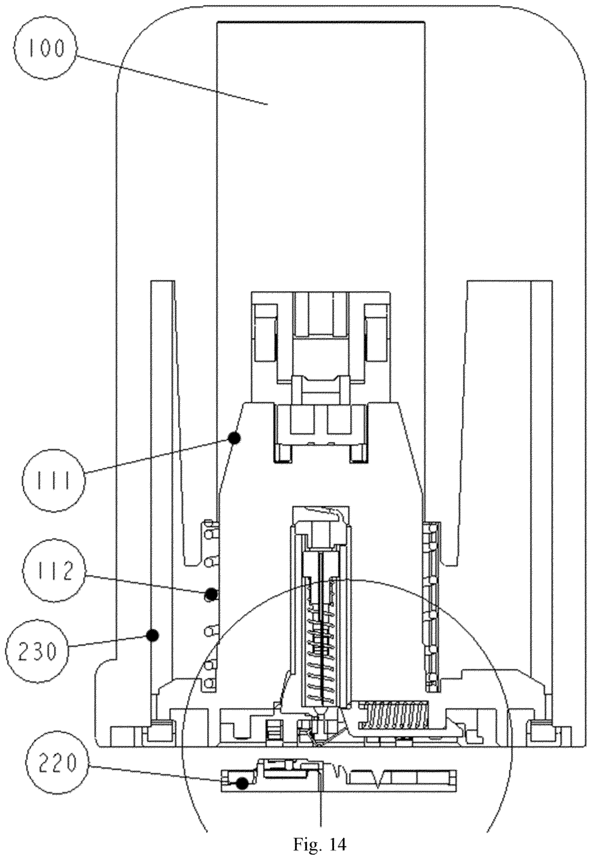

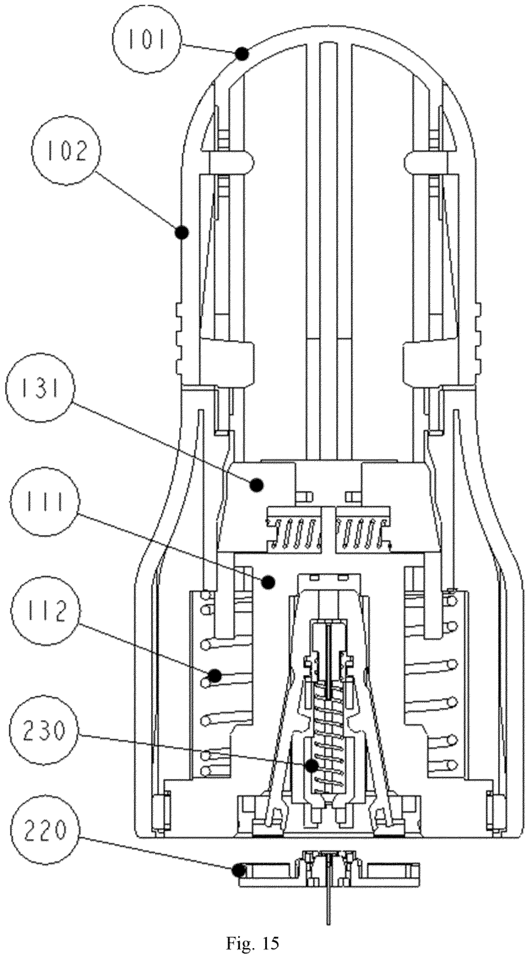

Referring to Figs. 14 to 16, the implantation device 100 is lifted in a direction directed toward the first end 101a (upwards, as viewed in the orientation of the figures). As the ejector assembly 111 is retained in the base body 101 by the fourth stoppers 1011 and the needle assembly 230 is retained in the ejector assembly 111 by the third stopper 141, the needle assembly 230 is lifted along with the implantation device 100.

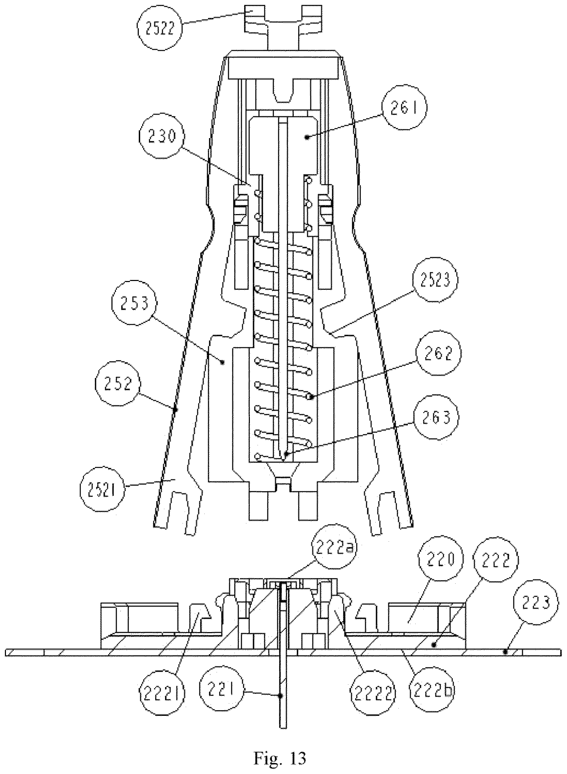

Since the adhesive layer 223 of the sensor base assembly 220 adheres to the skin surface, the sensor base assembly 220 is not lifted together with the sensor base assembly 220 and the needle assembly 230. Instead, the snap-engagement of the first snap engagement members 2521 and the second snap engagement members 2222, which attaches the sensor base assembly 220 to the needle assembly 230 (see Figs. 12 and 13), is destroyed, resulting in axial separation of the sensor base assembly 220 from the needle assembly 230.

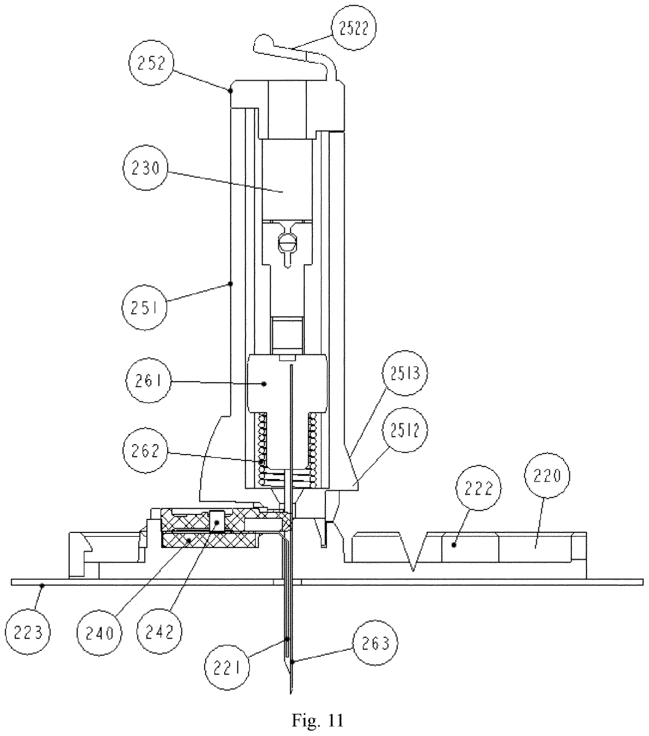

Further, referring to Fig. 13, during separation of a shell 251 of the needle assembly 230 (see Fig. 11) from the sensor base assembly 220, seventh potential energy storage elements 252 release potential energy that they have stored, releasing a needle body 263 from sixth stoppers 2523 which previously restricted movement of the needle body 263. Consequently, a sixth potential energy storage element 262 releases potential energy that it has stored, driving the needle body 263 to move away from an open end of the shell 251 (i.e., driving the needle body 263 to move upwards, as viewed in the orientation of Fig. 13). As a result, the needle body 263 is received in the shell 251, without possibly injuring an operator.

### <Assembly Step>

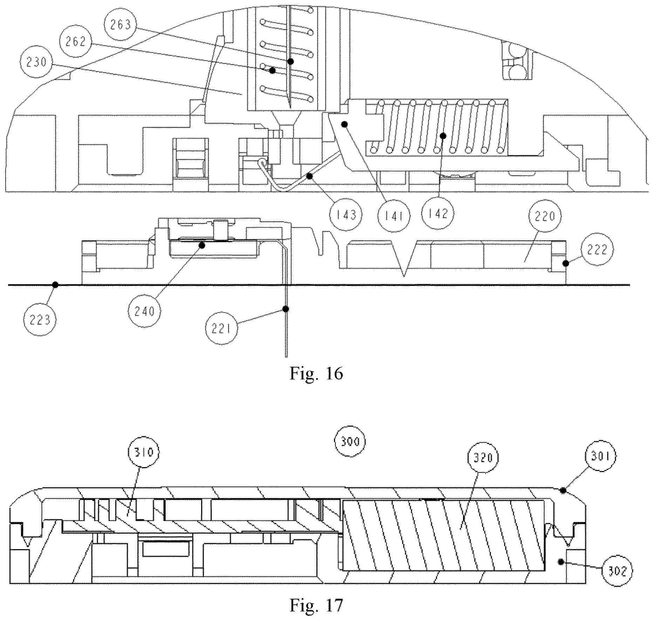

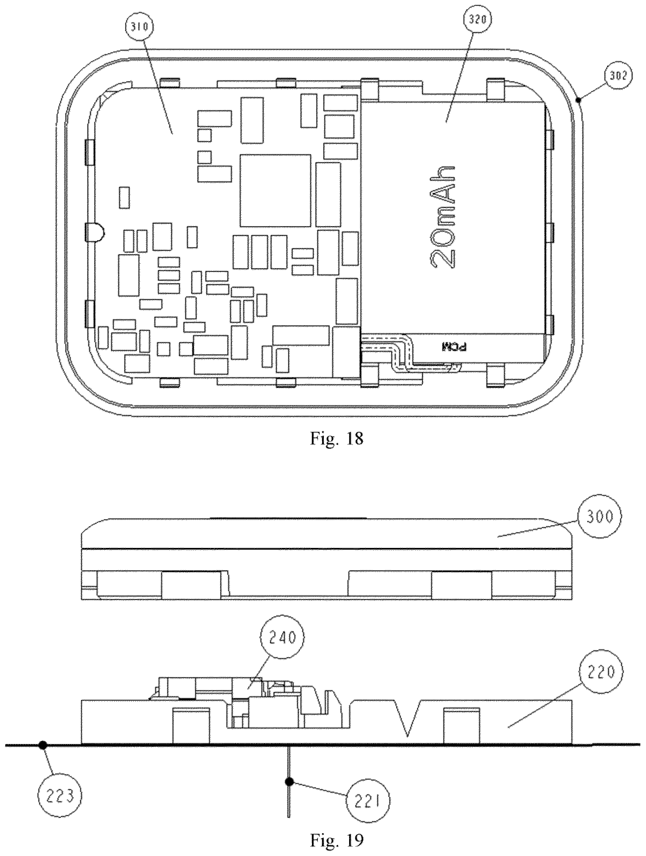

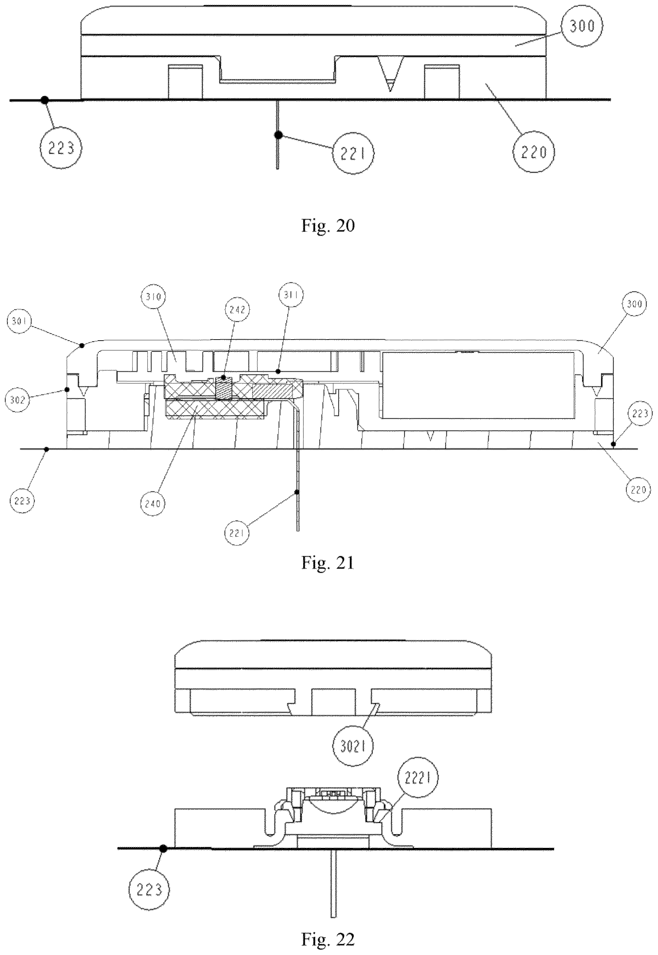

Referring to Figs. 17 to 21, after the sensor base assembly 220 is separated from the needle assembly 230, the emitter assembly 300 is assembled onto the sensor base assembly 220 so that the sensor component 221 of the sensor base assembly 220 is electrically connected to the emitter assembly 300. In this way, the sensor base assembly 220 is assembled with the emitter assembly 300.

### <Recovery Step>

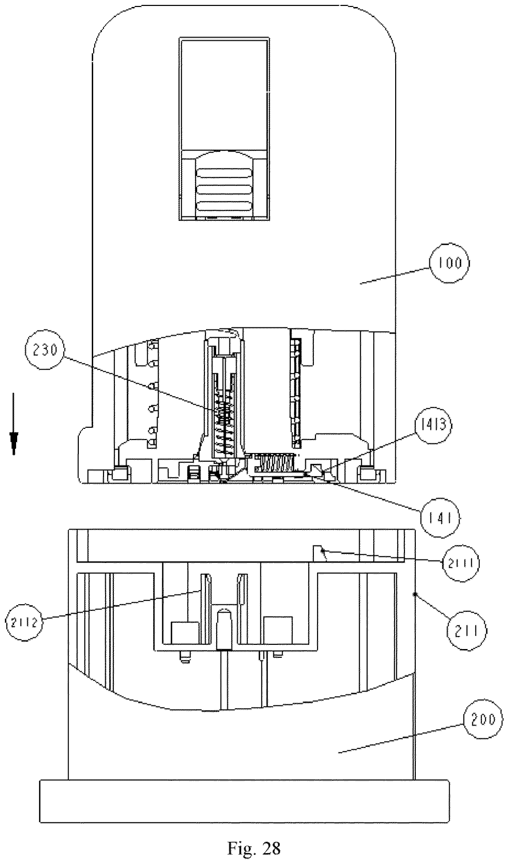

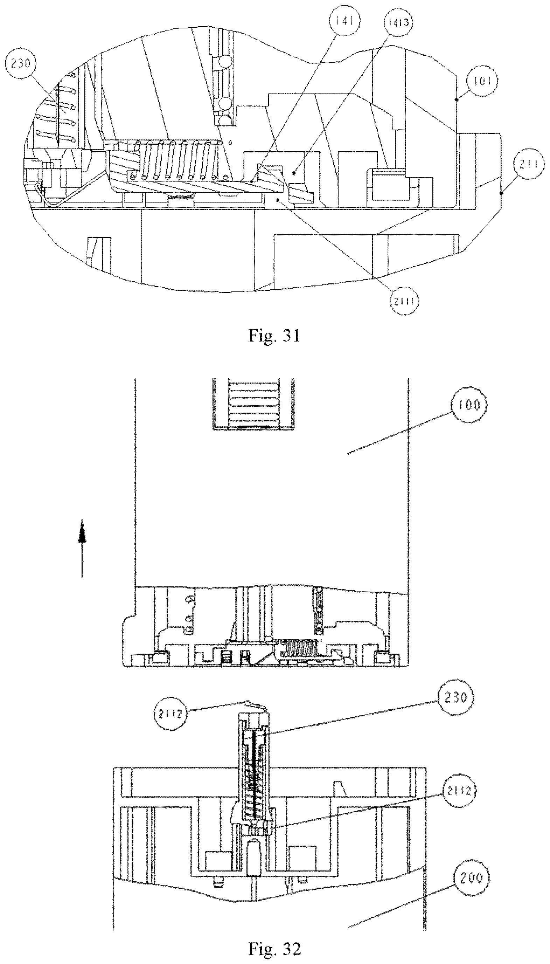

Referring to Figs. 28 to 31, after the sensor base assembly 220 is separated from the needle assembly 230, the sterilization box assembly 210 is turned upside down so that a needle recovery member 2111 projects upwards. The implantation device 100 is then moved towards the needle recovery member 2111 (downwards, as viewed in the orientation of Fig. 28) and inserted into the sterilization box assembly 210, causing the needle recovery member 2111 to urge the third stopper 141 away from the ejector assembly 111 (to the right, as viewed in the orientation of Fig. 31). As a result, the stop ledge 1412 of the third stopper 141 is moved away from abutment with the seventh stopper 2512 (see Fig. 11), and the needle assembly 230 drops out of the ejector assembly 111 under the action of gravity into the sterilization box assembly 210. In this way, the needle assembly 230 is withdrawn, and the implantation device 100 can be reused. It will be understood that this recovery step is optional.

Below, the various components of the implantation system are described in detail with reference to the accompanying drawings.

Referring to Fig. 2, in an optional exemplary embodiment, there is provided a sterilization box assembly 210 including a shell 211, a foil seal 212 and a top protective cover 213. The shell 211 defines a first internal cavity for accommodation of the assembled needle 230 and sensor base 220 which are assembled. The foil seal 212 is sealingly attached to the shell 211 (optionally with a hot melt adhesive, followed by sterilization by irradiation), closing the first cavity. The top protective cover 213 is attached to an inner surface of the foil seal 212 to restrict movement of a needle assembly 230 and a sensor base assembly 220. During use, the top protective cover 213 can be torn off along with the foil seal 212, exposing the assembled needle 230 and sensor base 220 assemblies in the first cavity. Of course, in some alternative embodiments, instead of receiving both the needle assembly 230 and the sensor base assembly 220 in the sterilization box assembly 210, only one of them may be received in the sterilization box assembly 210.

Referring to Figs. 3 to 6, embodiments of the present application provide an implantation device 100 including a base body 101, a press member 102, an ejector assembly 111 and a first potential energy storage element 112. The base body 101 has a first end 101a and a second end 101b, which oppose each other along an axis of the base body 101 (which extends vertically, as viewed in the orientation of Fig. 3). The base body 101 includes a first stopper 1014. The ejector assembly 111 is disposed so as to be movable along the axis of the base body 101. The ejector assembly 111 is configured for connecting with the needle assembly 230 and for detachable attachment to the sensor base assembly 220. During movement of the ejector assembly 111 toward the first end 101a, the first potential energy storage element 112 stores potential energy, and when the ejector assembly 111 moves and exceeds a first predetermined position (see Figs. 7 and 8, in which the ejector assembly 111 is shown as being located at the first predetermined position), it is restricted by the first stopper 1014 from moving toward the second end 101b. The ejector assembly 111 is released from the first stopper 1014 when the press member 102 is pressed down. Once this happens, the first potential energy storage element 112 releases potential energy that it has stored, causing the ejector assembly 111, together with the needle assembly 230 and the sensor base assembly 220, toward the second end 101b. In this way, the needle assembly 230 and the sensor base assembly 220 are ejected toward a predetermined implantation site. With this arrangement, the implantation device 100 and the needle assembly 230 that can be detached from the sensor base assembly 220 can be withdrawn after the implantation is completed, and the implantation device 100 can be reused. The only component that is implanted is the sensor base assembly 220, and the implantation device 100 can be recovered and reused many times, effectively reducing the cost and the influence on the environment. It should be noted that non-limiting examples of the first potential energy storage element 112 may include elastic potential energy storage elements (e.g., springs, resilient tabs, elastic plastic pieces, etc.) and magnetic potential energy storage elements (e.g., magnets, iron elements). In the exemplary embodiment shown in Figs. 3 and 4, the first potential energy storage element 112 includes a spring connected to the base body 101 at one end and to the ejector assembly 111 at the other end. When the ejector assembly 111 moves toward the first end 101a, the spring is compressed to store potential energy. In other embodiments, it is also possible to use a magnetic element capable of storing potential energy. Each of the second, third, fourth, fifth, sixth, seventh and eighth potential energy storage elements described below can store and release potential energy in any suitable similar manner as the first potential energy storage element 112. Reference can be made to the above description of the first potential energy storage element 112 for details of potential energy storage and release of those potential energy storage elements, and further description thereof is omitted hereinafter.

Referring to Figs. 6 and 7, in an optional exemplary embodiment, the base body 101 defines four elastic legs 1015, each of which extends along the axis of the base body 101 and has a free end proximal to the second end 101b. The elastic legs 1015 form two pairs each consisting of two elastic legs 1015, which are arranged in opposition to, and spaced apart from, each other to define a slide channel 1012 extending in parallel to the axis of the base body 101. Correspondingly, the ejector assembly 111 defines two sliders 1111, which are received in the two respective slide channels 1012 in such a manner that they do not have any other degree of freedom than that along the axis of the base body 101. In this way, restricted by the sliders 1111 and the slide channels 1012, the ejector assembly 111 can only move along the axis of the base body 101 without dislodging from the base body 101.

Optionally, the ejector assembly 111 includes a second stopper 131 and a second potential energy storage element 132. The second stopper 131 is disposed so as to be movable with respect to the base body 101 along the radial direction thereof. The second potential energy storage element 132 stores potential energy when the ejector assembly 111 moves in a direction from the second end 101b to the first end 101a. During movement of the ejector assembly 111 toward the first end 101a, when it moves and exceeds the first predetermined position, the second potential energy storage element 132 releases potential energy that it has stored, causing the second stopper 131 to move with respect to the base body 101 along the radial direction thereof to a second predetermined position (see Fig. 8, in which the second stopper 131 is shown as being located at the second predetermined position). The second stopper 131 is configured to, when at the second predetermined position, abut against the first stopper 1014, restricting the ejector assembly 111 from further moving toward the second end 101b. Referring to Fig. 4, in an exemplary embodiment, the second stopper 131 includes two stop blocks spaced apart from each other, and the second potential energy storage element 132 includes a spring disposed between the two stop blocks. The first stopper 1014 defines radially-extending ledges, and when the ejector assembly 111 moves toward the first end 101a and exceeds the first predetermined position, the spring of the second potential energy storage element 132 releases potential energy that it has stored, urging the two stop blocks of the second stopper 131 to move outwardly to the second predetermined position, where the stop blocks abut against the respective radially-extending flat ledges of the first stopper 1014. In this way, the ejector assembly 111 is restricted from moving toward the second end 101b and does not exceed the first predetermined position.

With the ejector assembly 111 being located at the first predetermined position and the second potential energy storage element 132 at the second predetermined position, when the press member 102 is pressed down, the second stopper 131 is driven to move with respect to the base body 101 along the radial direction thereof away from the second predetermined position. As a result, the second stopper 131 no longer abuts against the first stopper 1014. For example, the press member 102 may be provided on the base body 101 in such a manner that its one end is fixedly connected to the base body 101 and the other end is free. An operator may press the free end to cause the press member 102 to move with respect to the base body 101 along the radial direction thereof. An inner side of the press member 102 may abut against and drive the two stop blocks of the second stopper 131 to move inwardly until the stop blocks do not abut against the respective ledges of the first stopper 1014 any longer. Consequently, the spring of the first potential energy storage element 112 releases potential energy that it has stored, causing the ejector assembly 111 to be ejected instantly toward the second end 101b, so as to move together with the needle assembly 230 and the sensor base assembly 220 toward the predetermined implantation site (see Figs. 9 and 10).

Referring to Fig. 8, the base body 101 defines first sloped surfaces 1013 each sloped inwardly as it extends toward the second end 101b, and/or the second stopper 131 defines second sloped surfaces 1311 each sloped outwardly as it extends toward the first end 101a. While the ejector assembly 111 is moving toward the first end 101a, the second stopper 131 is urged by the first sloped surfaces 1013 and/or the second sloped surfaces 1311 to gradually move inwardly with respect to the base body 101 along the radial direction thereof, thereby causing the second potential energy storage element 132 to store potential energy.

It should be noted that, here, by "sloped inwardly as it extends toward the second end 101b", it is intended to mean that each first sloped surface 1013 is defined at an inner side of the base body 101 and faces toward the inside (i.e., the center axis) of the base body 101. Moreover, a normal to the first sloped surface 1013 points toward the second end 101b so that the first sloped surface 1013 is spaced at the side proximal to the second end 101b at a distance from the axis of the base body 101, which is greater than a distance that the first sloped surface 1013 is spaced from the axis of the base body 101 at the side proximal to the first end 101a.

The outward sloping of each second sloped surface 1311 in a direction toward the first end 101a can be construed with reference to the above definition of the inward sloping of each first sloped surface 1013 in a direction toward the second end 101b. Specifically, each second sloped surface 1311 is defined at an outer side of the second stopper 131 and faces toward the outside of the base body 101 (i.e., away from the center axis thereof). Moreover, a normal to the second sloped surface 1311 points toward the first end 101a so that the second sloped surface 1311 is spaced at the side proximal to the second end 101b at a distance from the axis of the base body 101, which is greater than a distance that the second sloped surface 1311 is spaced from the axis of the base body 101 at the side proximal to the first end 101a. Inward sloping and outward sloping of other components described in the following are interpreted in a similar manner, and further description thereof is omitted hereinafter.

In the exemplary embodiment shown in Fig. 4, the base body 101 defines two opposing first sloped surfaces 1013, and each of the two stop blocks of the second stopper 131 defines a second sloped surface 1311. The first sloped surfaces 1013 and the second sloped surfaces 1311 define upwardly tapered shapes. In particular, the first sloped surfaces 1013 and the second sloped surfaces 1311 may each be a flat or curved surface, or consist of several flat surface sections which are joined at angle(s), without departing from the scope of the present application. As would be appreciated, in some embodiments, only the first surfaces 1013 are sloped and the second sloped surfaces 1311 are parallel to the axis of the base body 101, or only the second sloped surfaces 1311 are sloped and the first surfaces 1013 are parallel to the axis of the base body 101, while still achieving the same results. Through providing the first sloped surfaces 1013 and/or the second sloped surfaces 1311, when the ejector assembly 111 moves toward the first end 101a, the two stop blocks of the second stopper 131 are urged to gradually move inwardly so as to drive the second potential energy storage element 132 to store potential energy.

Referring to Figs. 3, 4, 6 and 7, the base body 101 is optionally to include fourth stoppers 1011 for restricting the ejector assembly 111 from moving so as not exceed a fifth predetermined position (see Figs. 3 and 4, in which the ejector assembly 111 is shown as being located at the fifth predetermined position) during its movement toward the second end 101b. Optionally, the fourth stoppers 1011 are located at the ends of the elastic legs 1015 proximal to the second end 101b. For example, the fourth stoppers 1011 may be snap brackets provided on the elastic legs 1015, and their ends facing the first end 101a define stop ledges, which can abut against the sliders 1111 to restrict the ejector assembly 111 from moving toward the second end 101b so as not exceed the fifth predetermined position. In this way, the ejector assembly 111 will not dislodge from the base body 101.

In the implantation system shown in Fig. 1, in an initial configuration, both the needle assembly 230 and the sensor base assembly 220 are received in the sterilization box assembly 210. During use, the needle assembly 230 and the sensor base assembly 220 are together loaded into the ejector assembly 111 of the implantation device 100 so that the needle assembly 230 is detachably and movably attached to the ejector assembly 111. Referring to Figs. 7 and 8, the implantation device 100 is pushed down in an orientation with the second end 101b facing toward the needle assembly 230 and the sensor base assembly 220. Correspondingly, the needle assembly 230 and the sensor base assembly 220 move, along with the ejector assembly 111, toward the first end 101a, until the ejector assembly 111 reaches or passes the first predetermined position. In this process, the needle assembly 230 and the sensor base assembly 220 also move toward the first end 101a relative to the ejector assembly 111 until the needle assembly 230 reaches a third predetermined position (see Figs. 7 and 8, in which the needle assembly 230 is located at the third predetermined position relative to the ejector assembly 111). It is to be understood that the third predetermined position is a relative position of the needle assembly 230 to the ejector assembly 111. At this time, the ejector assembly 111 is not limited to being located at the first predetermined position. Rather, it may be located at a location closer to the first end 101a than the first predetermined position, or at any location between the first predetermined position and the fifth predetermined position.

Referring to Figs. 3 and 5, the ejector assembly 111 is optionally to include a third stopper 141 for restricting the needle assembly 230 from moving toward the second end 101b so as not exceed the third predetermined position relative to the ejector assembly 111. In an exemplary embodiment, the third stopper 141 defines a stop ledge 1412 facing the first end 101a, and the needle assembly 230 (more precisely, a seventh stopper 2512 of the needle assembly 230, as described in greater detail below) can abut against the stop ledge 1412 and is thereby restricted from moving toward the second end 101b and does not exceed the third predetermined position relative to the ejector assembly.

Further, the third stopper 141 is configured to be movable with respect to the base body 101 along the radial direction thereof. The ejector assembly 111 includes a third potential energy storage element 142 which stores potential energy when the needle assembly 230 moves relative to the ejector assembly 111 toward the first end 101a. When the needle assembly 230 moves toward the first end 101a and exceeds the third predetermined position relative to the ejector assembly, the third potential energy storage element 142 releases potential energy that it has stored, causing the third stopper 141 to move with respect to the base body 101 along the radial direction thereof to a fourth predetermined position (see Figs. 7 and 8, in which the third stopper 141 is shown as being located at the fourth predetermined position), where the third stopper 141 abuts against the needle assembly 230, thereby restricting the needle assembly 230 from moving toward the second end 101b aso as not exceed the third predetermined position relative to the ejector assembly 111.

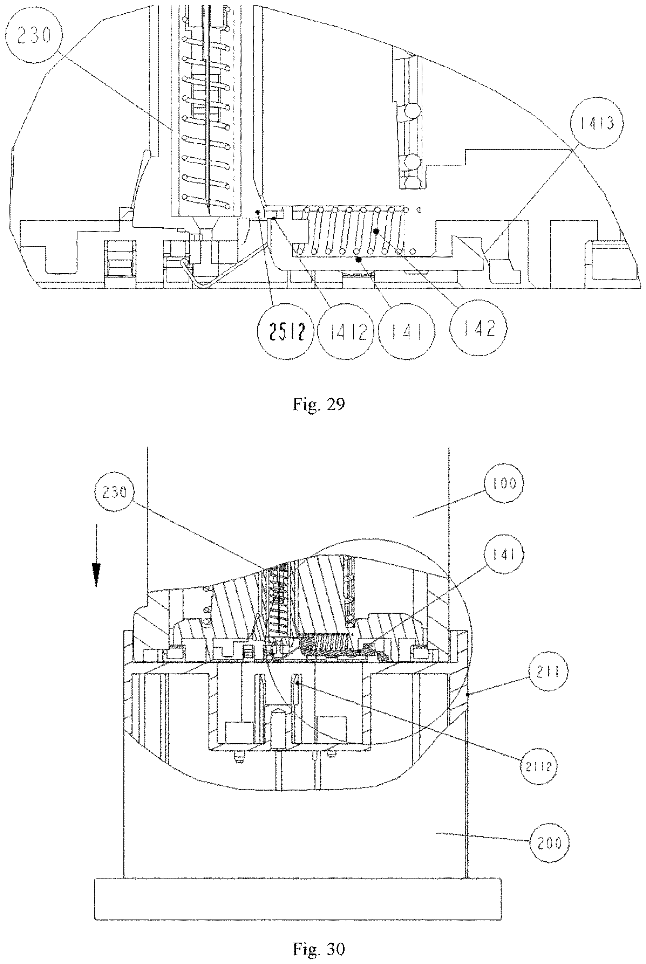

Optionally, the ejector assembly 111 may be detachably connected to the needle assembly 230. The third stopper 141 is configured so that when a needle recovery member 2111 (which is described in greater detail below with reference to Figs. 30 and 31) is brought into abutment with and acts upon it, the needle assembly 230 is released from it and can be separated from the ejector assembly 111. In some embodiments, the needle assembly 230 may be separated from the ejector assembly 111. Moreover, after the implantation is completed, the needle assembly 230 can be withdrawn from the ejector assembly 111 and replaced with a new needle assembly 230 for the implantation device.

Optionally, the third stopper 141 defines a third sloped surface 1411 which is sloped inwardly as it extends toward the second end 101b. When the needle assembly 230 moves relative to the ejector assembly 111 toward the first end 101a, the third sloped surface 1411 abuts against and acts on the needle assembly 230, causing the third stopper 141 to gradually move outwardly with respect to the base body 101 along the radial direction thereof. At the same time, the third potential energy storage element 142 is driven to store potential energy. The third sloped surface 1411 also serves to guide the needle assembly 230 to move toward the first end 101a. Referring to Figs. 5, 7 and 8, in an exemplary embodiment, the third potential energy storage element 142 includes a spring connected at one end to the third stopper 141. When the needle assembly 230 moves relative to the ejector assembly 111 toward the first end 101a, the third stopper 141 is urged by the third sloped surface 1411 to move outwardly (to the right, as viewed in the orientation of Figs. 5 and 7), and the spring of the third potential energy storage element 142 stores potential energy. When the needle assembly 230 moves so as to exceed the third predetermined position relative to the ejector assembly 111, the third stopper 141 is no longer pushed by the needle assembly 230, and the spring of the third potential energy storage element 142 releases potential energy that it has stored, urging the third stopper 141 to move inwardly (to the left, as viewed in the orientation of Figs. 5 and 7) to the fourth predetermined position, where the stop ledge 1412 of the third stopper 141 abuts against the seventh stopper 2512 of the needle assembly 230 (see Fig. 11), restricting the needle assembly 230 from moving toward the second end 101b so as not exceed the third predetermined position relative to the ejector assembly 111. In this way, the needle assembly 230 is confined within the ejection assembly 111, and will not dislodge from the ejector assembly 111.

In some other embodiments, the third stopper 141 may not be defined with the third sloped surface 1411. Instead, the seventh stopper 2512 of the needle assembly 230 may be defined with a sixth sloped surface 2513 (see Fig. 11), which is sloped outwardly as it extends toward the first end 101a. When the needle assembly 230 moves relative to the ejector assembly 111, the sixth sloped surface 2513 abuts against the third stopper 141 and urges it to move outwardly. Optionally, the third sloped surface 1411 and the sixth sloped surface 2513 may be both provided, because this can reduce resistance to the urging actions.

Of course, in some other embodiments, only the sensor base assembly 220 may be received in the sterilization box assembly 210, while the needle assembly 230 is instead provided in the ejector assembly 111. Further, in some embodiments, the needle assembly 230 may be fixedly or movably connected to the ejector assembly 111. In this case, the needle assembly 230 may be detachably connected to the sensor base assembly 220. During use, the needle assembly 230 moves toward the first end 101a, together with the ejector assembly 111. Moreover, it is ejected when the press member 102 is pressed down and then withdrawn together with the implantation device 100.

In case of the ejector assembly 111 being detachably connected to the sensor base assembly 220, since the connection between the needle assembly 230 and the sensor base assembly 220 is detachable and therefore tends to suffer from inadequate reliability, the ejector assembly 111 may be additionally provided with a fifth stopper 121 for ensuring a more reliable connection established between the ejector assembly 111 and the sensor base assembly 220 after the needle assembly 230 and the sensor base assembly 220 are loaded into the implantation device 100 (and when the ejector assembly 111 is located at the first predetermined position). On the other hand, the fifth stopper 121 is required to release the sensor base assembly 220 during movement of the ejector assembly 111 toward the second end 101b that occurs after the press member 102 is pressed down (the release may take place either when the ejector assembly 111 is located between the first and fifth predetermined positions, or when it is located at the fifth predetermined position) to allow the sensor base assembly 220 to be separated from the needle assembly 230 and the ejector assembly 111 once the ejector assembly 111 reaches the fifth predetermined position during its movement toward the second end 101b.

In order to achieve the connection between the ejector assembly 111 and the sensor base assembly 220 in different configuration, referring to Figs. 4, 8 and 10, the ejector assembly 111 optionally includes a fifth stopper 121, which is configured both to restrict the sensor base assembly 220 from moving toward the second end 101b relative to the ejector assembly 111 when the ejector assembly 111 is located at the first predetermined position and to release the sensor base assembly 220 during movement of the ejector assembly 111 from the first predetermined position toward the second end 101b. When the ejector assembly 111 is located at the first predetermined position, the fifth stopper 121 serves to additionally retain the sensor base assembly 220 to ensure that the sensor base assembly 220 will not dislodge from the implantation device 100 after the needle assembly 230 and the sensor base assembly 220 are loaded into the implantation device 100.

Referring to Fig. 8, in an optional exemplary embodiment, the base body 101 includes a fourth sloped surface 1016 which is sloped inwardly as it extends toward the second end 101b. The fifth stopper 121 is configured to be movable with respect to the base body 101 along the radial direction thereof. As the ejector assembly 111 moves toward the first end 101a, the fifth stopper 121 is urged by the fourth sloped surface 1016 to gradually move inwardly radially with respect to the base body 101 eventually into snap engagement with the sensor base assembly 220 (more precisely, a base 222 of the sensor base assembly 220, as described in greater detail below), thereby restricting the sensor base assembly 220 from moving toward the second end 101b relative to the ejector assembly 111. When the ejector assembly 111 moves toward the second end 101b, the fifth stopper 121 gradually moves outwardly with respect to the base body 101 along the radial direction thereof to be no longer in engagement with the sensor base assembly 220, thus releasing the sensor base assembly 220. Optionally, the fifth stopper 121 includes a fifth sloped surface 1211, which is outwardly sloped in a direction toward the first end 101a and configured to match in shape, and abut against, the fourth sloped surface.

The ejector assembly 111 also includes a fourth potential energy storage element 122 for providing, during movement of the ejector assembly 111 toward the second end 101b, energy required by the fifth stopper 121 to gradually move outwardly with respect to the base body 101 along the radial direction thereof. Referring to Figs. 4 and 6, in an optional exemplary embodiment, the fourth potential energy storage element 122 includes a spring coupled at one end to the fifth stopper 121. During movement of the ejector assembly 111 toward the first end 101a, the fifth stopper 121 is urged by a push force exerted by the fourth sloped surface 1016 and/or the fifth sloped surface 1211 to move inwardly (toward the middle, as viewed in the orientation of Fig. 4), and the spring of the fourth potential energy storage element 122 consequently stores potential energy. Upon the ejector assembly 111 reaching the first predetermined position during its movement toward the first end 101a, snap engagement members defined on an inner side of the fifth stopper 121 move into snap engagement with the base 222 of the sensor base assembly 220. During movement of the ejector assembly 111 toward the second end 101b, the spring of the fourth potential energy storage element 122 releases potential energy that it has stored, urging the fifth stopper 121 into abutment against the fourth sloped surface 1016 (or an inner wall surface of the base body 101 if the fourth sloped surface 1016 is not present). Subsequently, further movement of the ejector assembly 111 toward the second end 101b will cause gradual outward movement of the fifth stopper 121 (to opposite lateral sides, as viewed in the orientation of Fig. 4). Finally, the base 222 of the sensor base assembly 220 is disengaged from the fifth stopper 121.

Referring to Figs. 11 to 16, in an embodiment of the present application, there is provided a needle assembly 230 including a shell 251 and a needle body 263. The shell 251 is connected to the needle body 263 and is configured to be connected to the ejector assembly 111 in the implantation device. The shell 251 is also configured for detachable connection with the sensor base assembly 220. In an embodiment of the present application, there is provided a sensor base assembly 220 including a base 222 and an adhesive layer 223. The base 222 has a third end 222a and a fourth end 222b, which oppose each other along an axis of the base 222. The base 222 is configured for detachable connection with the shell 251 of the needle assembly 230 at the third end 222a. The adhesive layer 223 is provided at the fourth end 222b of the base 222. The needle assembly 230 can be assembled with the sensor base assembly 220 by the detachable connection of the shell 251 with the base 222. After the needle assembly 230 and the sensor base assembly 220 are ejected, along with the ejector assembly 111, toward a predetermined implantation site, the adhesive layer 223 of the sensor base assembly 220 will adhesively attach to the predetermined implantation site, and the needle assembly 230 will be separated from the sensor base assembly 220. Accordingly, the needle assembly 230 can be removed.

In an exemplary embodiment, the shell 251 includes seventh potential energy storage elements 252 and first snap engagement members 2521. The first snap engagement members 2521 are configured for detachable connection with the sensor base assembly 220 along the axis of the shell 251. Correspondingly, the base 222 of the sensor base assembly 220 includes second snap engagement members 2222 configured for detachable connection with the first snap engagement members 2521 along the axis of the shell 251. When the first snap engagement members 2521 are assembled with the sensor base assembly 220, the seventh potential energy storage elements 252 store potential energy and provide potential energy to the first snap engagement members 2521, which urges the first snap engagement members 2521 to move with respect to the shell 251 along the radial direction thereof into abutment against the sensor base assembly 220. The first snap engagement members 2521 may be, for example, concave notches or convex teeth. Correspondingly, the second snap engagement members 2222 for engagement with the first snap engagement members 2521 may be convex teeth or concave notches. It is to be understood that the first snap engagement members 2521 and the second snap engagement members 2222 optionally extend along the axis of the shell 251. That is, both the teeth and notches extend along the axis of the shell 251. This facilitates separation of the needle assembly 230 from the sensor base assembly 220. The engagement of the first snap engagement members 2521 and the second snap engagement members 2222 may be accomplished by friction or interference fits, which also facilitate separation.

In one embodiment, the seventh potential energy storage elements 252 are elastic legs, which may form part of the shell 251 and exhibit a certain degree of elasticity. The first snap engagement members 2521 is defined at end portions of the elastic legs facing toward the sensor base assembly 220. When the first snap engagement members 2521 are snap-engaged with the second snap engagement members 2222, the elastic legs of the seventh potential energy storage elements 252 store potential energy. At the same time, the seventh potential energy storage elements 252 outwardly (to opposite lateral sides, as viewed in the orientation of Fig. 12) exert forces on the second snap engagement members 2222 through the first snap engagement members 2521, which radially urge the first snap engagement members 2521 into abutment against the second snap engagement members 2222 and create friction therebetween. In this way, the needle assembly 230 is assembled with the sensor base assembly 220. In order to separate the needle assembly 230 from the sensor base assembly 220, an axial force is applied to overcome the friction between them to cause them to move relative to each other in opposite direction. As a result of the separation, the elastic legs release potential energy that they have stored and deflect outwardly (to opposite lateral sides, as viewed in the orientation of Fig. 13).

In some embodiments, the needle body 263 may be fixed within the shell 251 and move along therewith. In some alternative embodiments, the needle body 263 is coupled to the shell 251 so as to be movable along the axis thereof.

Referring to Fig. 12, optionally, the needle assembly further includes a needle sheath 253 and a sixth potential energy storage element 262. Opposite ends of the sixth potential energy storage element 262 are fixed respectively to the needle sheath 253 and the needle body 263. When the shell 251 is assembled with the sensor base assembly 220, the sixth potential energy storage element 262 stores potential energy and the needle body 263 protrudes out of the needle sheath 253 along the axis of the shell 251. When the shell 251 is separated from the sensor base assembly 220, the sixth potential energy storage element 262 releases potential energy that it has stored, and the needle body 263 gradually retracts into the needle sheath 253 along the axis of the shell 251, avoiding possible accidental puncture or injury.

As shown in Fig. 13, in an optional exemplary embodiment, the needle body 263 is connected to the shell 251 so as to be movable along the axis thereof, while the needle sheath 253 is fixedly connected to the shell 251. When the shell 251 is assembled with the sensor base assembly 220, the sixth potential energy storage element 262 stores potential energy, and the needle body 263 protrudes out of the needle sheath 253 along the axis of the shell 251. When the shell 251 is separated from the sensor base assembly 220, the sixth potential energy storage element 262 releases potential energy that it has stored, retracting the needle body 263 back into the needle sheath 253. In this exemplary embodiment, since the needle sheath 253 is fixed to the shell 251, when the shell 251 is separated from the sensor base assembly 220, the sixth potential energy storage element 262 retracts the needle body 263 back (upwards, as viewed in the orientation of Fig. 13) into the needle sheath 263.

As shown in Fig. 12, in another optional exemplary embodiment, the needle body 263 is fixedly connected to the shell 251, while the needle sheath 253 is connected to the shell 251 so as to be movable along the axis thereof. When the shell 251 is assembled with the sensor base assembly 220, the sixth potential energy storage element 262 stores potential energy, and the needle sheath 253 is blocked by the shell 251 so that the needle body 263 protrudes out of the needle sheath 253. When the shell 251 is separated from the sensor base assembly 220, the shell 251 releases the needle sheath 253, and the sixth potential energy storage element 262 releases potential energy that it has stored, gradually advancing the needle sheath 253 over the needle body 263 along the axis of the shell 251. That is, the needle sheath 253 is moved and eventually sleeved over the needle body 263. In this exemplary embodiment, since the needle body 263 is fixed to the shell 251, when the shell 251 is separated from the sensor base assembly 220, the sixth potential energy storage element 262 ejects the needle sheath 253 (downwards as viewed in the orientation of Fig. 12) so that the needle body 263 is received in the needle sheath 253.

The shell 251 also includes sixth stoppers 2523, which are configured to abut against the needle body 263 or the needle sheath 253 (whichever is movable, depending on different embodiments) when the shell 251 is assembled with the sensor base assembly 220, thereby allowing the sixth potential energy storage element 262 to store potential energy and restricting axial position of the needle body 263 or the needle sheath 253 with respect to the shell 251. When the shell 251 is separated from the sensor base assembly 220, the sixth stoppers 2523 move away from abutment against the needle body 263 or the needle sheath 253. Consequently, the sixth potential energy storage element 262 releases potential energy that it has stored, and the needle body 263 or the needle sheath 253 is no longer restricted from movement. For example, the sixth stoppers 2523 may be inward projections on the elastic legs of the seventh potential energy storage elements 252. When the needle assembly 230 is assembled with the sensor base assembly 220, the first snap engagement members 2521 are in snap-engagement with the second snap engagement members 2222, and the elastic legs of the seventh potential energy storage elements 252 are compressed inwardly so that the sixth stoppers 2523 are kept in abutment against the needle body 263 or the needle sheath 253.

In the exemplary embodiment where the needle sheath 253 is fixed while the needle body 263 is movable, the sixth stoppers 2523 are configured to restrict the needle body 263 from moving away from the sensor base assembly 220 (upwards, as viewed in the orientation of Fig. 12). Moreover, when the needle assembly 230 is separated from the sensor base assembly 220, the first snap engagement members 2521 are disengaged from the second snap engagement members 2222, and the seventh potential energy storage elements 252 release potential energy that they have stored. As a result, the elastic legs deflect outwardly, moving the sixth stoppers 2523 away from abutment against the needle body 263. Consequently, the sixth potential energy storage element 262 releases potential energy that it has stored, retracting the needle body 263 upwards, as viewed in the orientation of Fig. 13, from the predetermined implantation site back into the needle sheath 253 at a very fast speed and thereby avoiding potential accidental injury. It would be appreciated that, in this exemplary embodiment, the retraction of the needle body 263 may occur in an automatic manner. That is, as a result of the shell 251 being separated from the sensor base assembly 220, the needle body 263 is automatically retracted back. Of course, in this exemplary embodiment, the retraction may also occur as a result of an operator lifting the implantation device 100.

In the exemplary embodiment where the needle body 263 is fixed while the needle sheath 253 is movable, the sixth stoppers 2523 are configured to restrict the needle sheath 253 from moving downwards, as viewed in the orientation of Fig. 12. Moreover, when the needle assembly 230 is separated from the sensor base assembly 220, the first snap engagement members 2521 are disengaged from the second snap engagement members 2222, and the seventh potential energy storage elements 252 release potential energy that they have stored. As a result, the elastic legs deflect outwardly, moving the sixth stoppers 2523 away from abutment against the needle sheath 253. Consequently, the sixth potential energy storage element 262 releases potential energy that it has stored, ejecting the needle sheath 253 downwards, as viewed in the orientation of Fig. 12, so that the needle sheath 253 is sleeved over the needle body 263 and thereby avoiding possible accidental injury. It would be appreciated that, in this exemplary embodiment, the ejection of the needle sheath 253 occurs in a semi-automatic manner. That is, the needle sheath 253 cannot be sleeved over the needle body 263 immediately after the shell 251 is separated from the sensor base assembly 220 because its lower end is blocked by the sensor base assembly 220 and a human body. At this time, the operator needs to lift the implantation device 100 to withdraw the needle body 263 from the human body. Only when there is some space under the needle assembly 230, can the needle sheath 253 be ejected so as to be sleeved over the needle body 263.

Optionally, the needle body 263 includes a steel needle for piercing the skin at the implantation site and a needle holder 261 disposed at an end of the steel needle (the upper end, as viewed in the orientation of Fig. 13). The steel needle is secured to the needle holder 261. The sixth potential energy storage element 262 may be, for example, a spring, one end of which is connected to the needle holder 261, and the other end to the needle sheath 253. The sixth potential energy storage element 262 is provided to cause the needle body 263 to be received back into the needle sheath 253 when the needle assembly 230 is separated from the sensor base assembly 220. According to the present application, the needle assembly 230 may be configured so that the needle body is retracted after a sensor component has been successfully implanted at the predetermined site, either automatically or as a result of withdrawing the implantation device.

Referring to Fig. 11, optionally, the shell 251 includes a seventh stopper 2512 configured to abut against the third stopper 141 of the ejector assembly 111 (see Fig. 5), restricting the shell 251 from moving toward the second end 101b so as not exceed the third predetermined position relative to the ejector assembly 111. When the needle assembly 230 is assembled with the sensor base assembly 220, the seventh stopper 2512 defines a radial size smaller than a radial size defined by the seventh potential energy storage elements 252. Therefore, it can abut against the third stopper 141 of the ejector assembly 111 to restrict the shell 251 from dislodging from the ejector assembly 111 as the needle body 263 is piercing the skin at the pierce implantation site. Optionally, the seventh stopper 2512 defines a sixth sloped surface 2513, which is sloped outwardly as it extends toward the first end 101a. Further, the needle assembly 230 is detachably connected to the ejector assembly 111, and when the seventh stopper 2512 is moved away from abutment against the third stopper 141 (e.g., as a result of a needle recovery member 2111 being brought into abutment against the third stopper 141), the shell 251 becomes movable again and the needle assembly 230 is detached from the ejector assembly 111.

Referring to Figs. 5, 14 and 15, optionally, the ejector assembly 111 further includes a fifth potential energy storage element 143 which stores potential energy when the ejector assembly 111 is assembled with the sensor base assembly 220. Moreover, when the ejector assembly 111 is separated from the sensor base assembly 220, the fifth potential energy storage element 143 releases potential energy that it has stored, causing the sensor base assembly 220 to move away from the ejector assembly 111. The fifth potential energy storage element 143 may be, for example, a flat spring which is compressed to store potential energy when the needle assembly 230 and the sensor base assembly 220 are both assembled onto the ejector assembly 111. Moreover, when the sensor base assembly 220 is separated from the needle assembly 230, the flat spring of the fifth potential energy storage element 143 releases potential energy that it has stored, urging the sensor base assembly 220 to move away from the needle assembly 230 and facilitating their separation from each other. Specifically, when the sensor base assembly 220 and the needle assembly 230 are ejected along with the ejector assembly 111, the adhesive layer 223 attaches the sensor base assembly 220 to the predetermined implantation site. At this point, the implantation device 100 is pulled away from the predetermined implantation site to allow the fifth potential energy storage element 143 to release potential energy that it has stored to facilitate separation of the sensor base assembly 220 from the needle assembly 230. It would be appreciated that the potential energy that the fifth potential energy storage element 143 can store should not be enough to overcome the friction between the first snap engagement members 2521 in the seventh potential energy storage elements 252 and the second snap engagement members 2222, in order to enable the sensor base assembly 220 and the needle assembly 230 to be assembled with each other with sufficient stability. In some embodiments, after the needle assembly 230 and the sensor base assembly 220 are ejected by the implantation device 100 toward the predetermined implantation site and the needle assembly 230 is separated from the sensor base assembly 220, the needle assembly 230 is remained in the implantation device 100. After that, some action may be taken to remove the needle assembly 230 from the implantation device 100, and optionally, the needle assembly 230 is then placed into the sterilization box assembly 210.

Referring to Figs. 28 to 32, optionally, the sterilization box assembly 210 includes a needle recovery member 2111 configured to abut against the third stopper 141 of the implantation device 100. When this happens, the third stopper 141 will be urged by the needle recovery member 2111 to release the needle assembly 230 and allow it to move toward the second end 101b, resulting in separation of the needle assembly 230 from the implantation device 100. Further, in some embodiments, the shell 211 of the sterilization box further defines a second cavity, for example, at the side of the shell 211 axially opposite to the first cavity. The needle recovery member 2111 is provided at the side of the shell 211 of the sterilization box opposite to the first cavity. After the needle assembly 230 is released from the third stopper 141 of the implantation device 100 as a result of the needle recovery member 2111 being in abutment against the third stopper 141, the needle assembly 230 moves from the implantation device 100 into the second cavity.

In an exemplary embodiment, the third stopper 141 serves to support the needle assembly 230, facilitate separation of the needle assembly 230 from the sensor base assembly 220 and subsequently separate the needle assembly 230 from the implantation device 100. Specifically, referring to Fig. 29, one end of the third stopper 141 (the left end, as viewed in the orientation of Fig. 29) is brought into abutment with the seventh stopper 2512 of the needle assembly 230 to restrict movement of the needle assembly 230. The third stopper 141 may be overall movable with respect to the base body 101 along the radial direction thereof (i.e., the left-right direction, as viewed in the orientation of Fig. 29), and the left end of the third stopper 141 has a bent portion extending along the axis of the base body 101. An upper end of the bent portion defines a stop ledge 1412 configured to abut against the seventh stopper 2512 to restrict the needle assembly 230 from dislodging from the ejector assembly 111. A right end of the bent portion is connected to the base body 101 through the third potential energy storage element 142. With this arrangement, when there is no external force applied to the third stopper 141, the bent portion will be maintained in an extended position where it blocks the needle assembly 230.

The other end of the third stopper 141 (the right end, as viewed in the orientation of Fig. 29) defines a stop surface 1413 oriented toward the inside of the ejector assembly 111 (to the left, as viewed in the orientation of Fig. 29). The needle recovery member 2111 may be provided at the side opposite to the first cavity in the shell 211 of the sterilization box, particularly at the side opposite to the foil seal 212, i.e., at the bottom of the sterilization box assembly 210, in the example shown in Fig. 32. During use, the sterilization box assembly 210 may be turned upside down so that the needle recovery member 2111 projects upwards.

Referring to Figs. 31 and 32, during the process of the implantation device 100 being moved toward the bottom of the sterilization box assembly 210 and inserted into the sterilization box assembly 210, the needle recovery member 2111 moves the third stopper 141 outwardly from the ejector assembly 111 (to the right, as viewed in the orientation of Fig. 31), displacing the stop ledge 1412 of the third stopper 141 away from abutment against the seventh stopper 2512. Accordingly, the needle assembly 230 may drop under the action of gravity from the ejector assembly 111 into the sterilization box assembly 210. Optionally, the sterilization box assembly 210 further includes a retaining receptacle 2112 provided at the bottom of the sterilization box assembly 210 in alignment with the needle assembly 230 when it is falling, as shown in Figs. 28 and 32. Accordingly, in order to separate the needle assembly 230 from the implantation device 100, the sterilization box assembly 210 is turned upside down, and the needle recovery member 2111 pulls the ejector assembly 111 to release the needle assembly 230 from the ejector assembly 111. As a result, the needle assembly 230 will drop onto the retaining receptacle 2112.

Optionally, the needle assembly 230 further includes an eighth potential energy storage element 2522 (see Figs. 11 to 13). When the shell 251 is assembled with the ejector assembly 111, the eighth potential energy storage element 2522 stores potential energy. When the shell 251 is separated from the ejector assembly 111, the eighth potential energy storage element 2522 releases potential energy that it has stored, causing the shell 251 to move along an axis of the ejector assembly 111 and thereby separating it from the ejector assembly 111. The eighth potential energy storage element 2522 may be, for example, an elastic element configured to push, when the needle assembly 230 is separated from the ejector assembly 111, the needle assembly 230 in the opposite direction to facilitate removal of the needle assembly 230 from the ejector assembly 111, and to facilitate unloading of the needle assembly.

In the implantation system as discussed above, after the needle assembly 230 is assembled with the sensor base assembly 220, the resulting assembly is placed in the sterilization box assembly 210. The implantation device 100 is then inserted in the direction from the first end 101a to the second end 101b into the assembly of the needle assembly 230 and the sensor base assembly 220 in the sterilization box assembly 210 until the ejector assembly 111 reaches the first predetermined position during its movement toward the first end 101a. After the sensor base assembly 220 is attached to the predetermined implantation site, the needle assembly 230 is separated from the sensor base assembly 220, and the emitter assembly 300 is assembled with the sensor base assembly 220 after the needle assembly 230 is separated from the sensor base assembly 220.

Referring to Figs. 17 to 24, optionally, the base 222 is detachably connected at the third end 222a to the emitter assembly 300, and the sensor base assembly 220 further includes a sensor component 221 which is configured so as to be electrically connected to the emitter assembly 300 as a result of the sensor base assembly 220 being connected to the emitter assembly 300 after the needle body 263 of the needle assembly 230 is implanted at the predetermined site. The emitter assembly 300 may acquire monitored data from the sensor component 221 and emit the monitored data to any device that requires it. Optionally, the emitter assembly 300 includes a top housing 301, a bottom housing 302, a circuit board 310 and a battery 320. The circuit board 310 and the battery 320 are accommodated between the top housing 301 and the bottom housing 302, and the battery 320 supplies the circuit board 310 with power.

Optionally, the base 222 includes a third snap engagement member 2221 configured for snap engagement with the emitter assembly 300. Further, in an embodiment of the present application, there is also provided an emitter assembly 300 including a fourth snap engagement member 3021 configured for snap engagement with the third snap engagement member 2221 in the sensor base assembly 220. The third snap engagement member 2221 and the fourth snap engagement member 3021 may be a mating pair of male and female snap fasteners, such as a hook and a slot. After the adhesive layer 223 attaches the sensor base assembly 220 to the predetermined implantation site and the implantation device 100 is withdrawn, the emitter assembly 300 may be assembled onto the sensor base assembly 220 so that the third snap engagement member 2221 is snap-engaged with the fourth snap engagement member 3021. This can ensure reliable assembly of the emitter assembly 300 with the sensor base assembly 220.

More optionally, the third snap engagement member 2221 may be arranged at either the periphery or the interior of the base 222. In the former case, the emitter assembly 300 is assembled onto the sensor base assembly 220 simply by pressing down the emitter assembly 300 once the third snap engagement member 2221 is aligned with the fourth snap engagement member 3021. When the third snap engagement member 2221 is arranged at the interior of the base 222, the emitter assembly 300 is tilted at an angle (e.g., about 30 degrees) to allow a snap plug projecting from its bottom to be inserted into a snap recess defined in the sensor base assembly 220. After that, the other side of the titled emitter assembly 300 is pressed to engage the third snap engagement member 2221 with the fourth snap engagement member 3021 which is disposed within the snap recess of the sensor base assembly 220. Both these two approaches allow reliable assembly of the emitter assembly 300 with the sensor base assembly 220. Further, in the second approach, since the assembly occurs at the interior of the base 222, lever principle can be applied to achieve more labor-saving assembly.

Optionally, the sensor base assembly 220 further includes a flexible conductor 242, which is electrically connected to the sensor component 221 and configured for detachable connection to the emitter assembly 300. The flexible conductor 242 may be, for example, an electrically conductive adhesive tape which is elastically preloaded between the sensor component 221 and the circuit board 310 in the emitter assembly 300, to electrically connect them to each other, as a result of the third snap engagement member 2221 being snap-engaged with the fourth snap engagement member 3021.

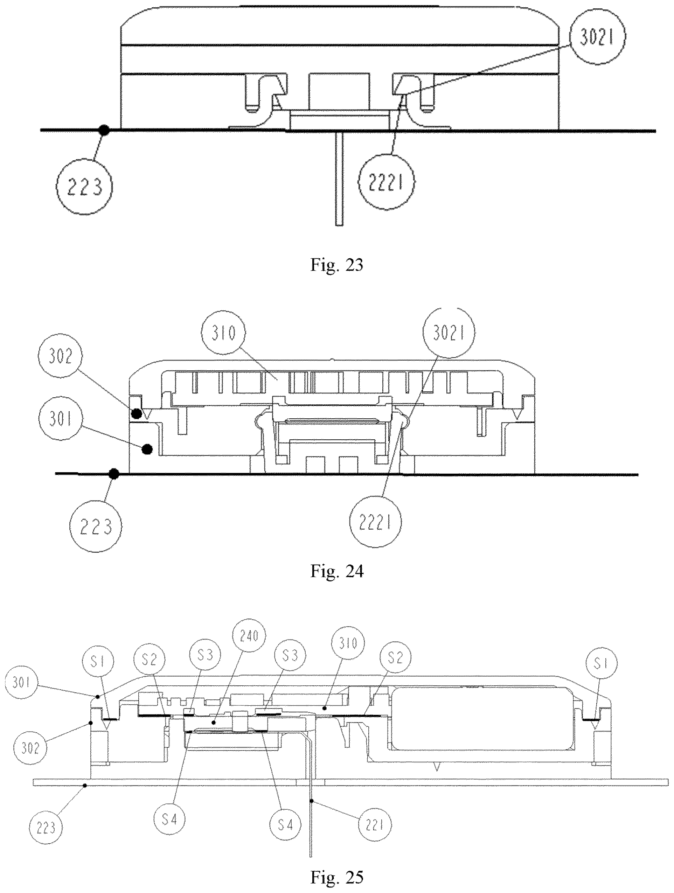

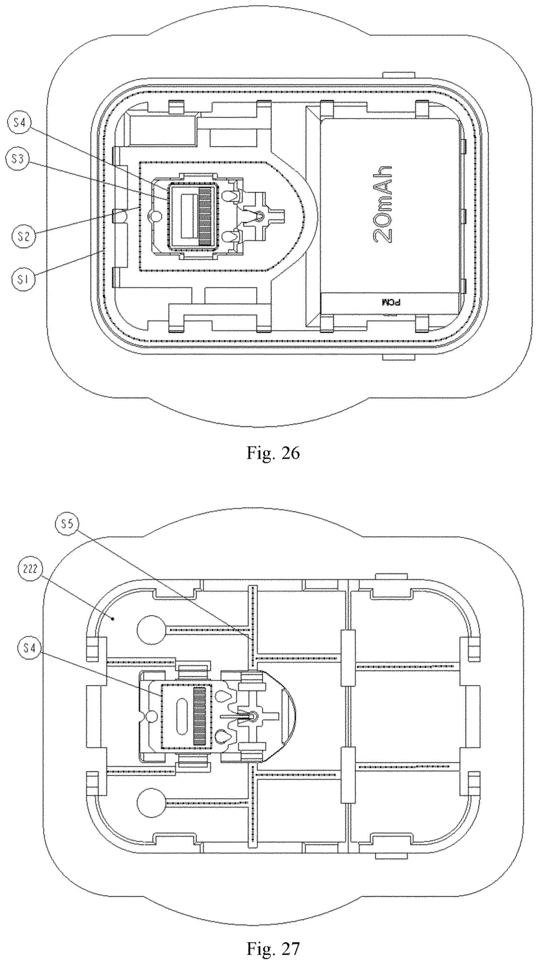

Optionally, an overall waterproof design is employed for the emitter assembly 300 and the sensor base assembly 220. Referring to Figs. 25 to 27, in an exemplary embodiment, the sensor base assembly 220 further includes an elastic seal 240 made of, for example, silicone or the like.

The overall waterproof design includes, but is not limited to:
S1) the top housing 301 being sealed to the bottom housing 302 along the entire periphery, e.g., by ultrasonic welding, a rubber ring, integral injection molding, etc.;
S2) the circuit board 310 being placed between the top housing 301 and the bottom housing 302 and completely sealed to a peripheral edge of a depression defined in the bottom housing 302 using an adhesive or integral injection molding;
S3) an upper surface of the elastic seal 240 being sealed to the periphery of an exposed middle portion of the circuit board 310 by squeezing them against each other with an elastic preload;
S4) the entire sensor component 221, except for the portion covered by the flexible conductor 242, being completely wrapped by the elastic seal 240, and an elastic preload being applied to squeeze them against each other for sealing;
S5) a drainage groove defined in a portion of the base 222 where it is brought into contact with the bottom of the emitter assembly 300, which can additionally enhance waterproof sealing.

In summary, the present application provides an implantation device, a needle assembly, a sensor base assembly, an emitter assembly, a sterilization box assembly and an implantation system. The implantation device includes a base body, a press member, an ejector assembly and a first potential energy storage element. The base body has axially opposing first and second ends and includes a first stopper. The ejector assembly is disposed so as to be movable along the axis of the base body. Moreover, the ejector assembly is configured to be connected to the needle assembly and detachably connected to the sensor base assembly. The first potential energy storage element stores potential energy as the ejector assembly moves toward the first end. Once the ejector assembly moves toward the first end and exceeds a first predetermined position, it is restricted by the first stopper from moving toward the second end. When the press member is pressed down, the ejector assembly is released from the first stopper, and the first potential energy storage element releases potential energy that it has stored, causing the ejector assembly, along with the needle assembly and the sensor base assembly, to move toward the second end.

With this arrangement, when the press member is pressed down, the first potential energy storage element releases potential energy that it has stored, causing the ejector assembly to move, along with the needle assembly and the sensor base assembly, so that the implantation is completed. Moreover, since the ejector assembly is detachable from the sensor base assembly, after the implantation is completed, the implantation device and the needle assembly can be both removed. What is to be implanted is only the sensor base assembly, and after the needle assembly is removed, the implantation device can be reused many times, effectively reducing the cost and the influence on the environment.

It is to be noted that the foregoing several embodiments may be combined. The description presented above is merely that of a few preferred embodiments of the present application and is not intended to limit the scope thereof in any sense. Any and all changes and modifications made by those of ordinary skill in the art based on the above teachings fall within the scope as defined in the appended claims.

## Claims

1. An implantation device, comprising a base body, a press member, an ejector assembly and a first potential energy storage element,
the base body having a first end and a second end, which oppose each other along an axis of the base body, the base body comprising a first stopper, the ejector assembly disposed so as to be movable along the axis of the base body, the ejector assembly configured to be connected to a needle assembly and detachably connected to a sensor base assembly,
the first potential energy storage element configured to store potential energy as the ejector assembly moves toward the first end, the ejector assembly configured to be restricted by the first stopper from moving toward the second end once the ejector assembly moves toward the first end and exceeds a first predetermined position,
when the press member is pressed, the ejector assembly is released from the first stopper, and the first potential energy storage element releases potential energy to cause the ejector assembly to move toward the second end along with the needle assembly and the sensor base assembly.

2. The implantation device according to claim 1, wherein the ejector assembly comprises a second stopper and a second potential energy storage element,
the second stopper disposed so as to be movable along a radial direction of the base body,
the second potential energy storage element configured to store potential energy as the ejector assembly moves in a direction from the second end to the first end and release potential energy, when the ejector assembly moves toward the first end and exceeds the first predetermined position, to cause the second stopper to move with respect to the base body along the radial direction of the base body to a second predetermined position, wherein the second stopper, when at the second predetermined position, is configured to abut against the first stopper to restrict the ejector assembly from moving toward the second end.

3. The implantation device according to claim 2, wherein with the ejector assembly being located at the first predetermined position and the second stopper at the second predetermined position, when the press member is pressed, the second stopper is caused to move along the radial direction of the base body away from the second predetermined position so that the second stopper does not abut against the first stopper.

4. The implantation device according to claim 2 or 3, wherein the base body comprises a first sloped surface which is sloped inwardly as extending toward the second end, and/or the second stopper comprises a second sloped surface which is sloped outwardly as extending toward the first end,
wherein during movement of the ejector assembly toward the first end, the second stopper is restricted by the first sloped surface and/or the second sloped surface to gradually move inwardly along the radial direction of the base body so that the second potential energy storage element is caused to store potential energy.

5. The implantation device according to claim 1, wherein the ejector assembly is configured to be movably connected to the needle assembly and comprises a third stopper configured to restrict the needle assembly from moving toward the second end so as not exceed a third predetermined position relative to the ejector assembly.

6. The implantation device according to claim 5, wherein the third stopper is disposed so as to be movable radially with respect to the base body, wherein the ejector assembly comprises a third potential energy storage element configured to store potential energy as the needle assembly moves toward the first end relative to the ejector assembly,
when the needle assembly moves toward the first end and exceeds the third predetermined position relative to the ejector assembly, the third potential energy storage element releases potential energy to cause the third stopper to move along a radial direction of the base body to a fourth predetermined position, and wherein the third stopper, when at the fourth predetermined position, is configured to abut against the needle assembly to restrict the needle assembly from moving toward the second end so as not exceed the third predetermined position relative to the ejector assembly.

7. The implantation device according to claim 6, wherein the third stopper comprises a third sloped surface which is sloped inwardly as extending toward the second end,
wherein as the needle assembly moves toward the first end relative to the ejector assembly, with the third sloped surface abutting against the needle assembly, the third stopper moves outwardly along the radial direction of the base body to cause the third potential energy storage element to store potential energy.

8. The implantation device according to claim 5, wherein the ejector assembly is detachably connected to the needle assembly, wherein the third stopper is configured to release the needle assembly when abutting against a needle recovery member so that the needle assembly is separated from the ejector assembly.

9. The implantation device according to claim 1, wherein the base body comprises a fourth stopper configured to restrict the ejector assembly from moving toward the second end so as not exceed a fifth predetermined position.

10. The implantation device according to claim 1, wherein the ejector assembly comprises a fifth stopper,
when the ejector assembly is located at the first predetermined position, the fifth stopper restricts the sensor base assembly from moving toward the second end relative to the ejector assembly,
wherein the fifth stopper less restricts the sensor base assembly as the ejector assembly moves from the first predetermined position toward the second end.

11. The implantation device according to claim 10, wherein the base body comprises a fourth sloped surface which is sloped inwardly as extending toward the second end,
wherein the fifth stopper is disposed so as to movable along a radial direction of the base body;
as the ejector assembly moves toward the first end, the fifth stopper is restricted by the fourth sloped surface to gradually move inwardly along the radial direction of the base body into snap-engagement with the sensor base assembly so that the sensor base assembly is restricted from moving toward the second end relative to the ejector assembly; and
as the ejector assembly moves toward the second end, the fifth stopper gradually moves outwardly along the radial direction of the base body so that pressure of snap-engagement between the fifth stopper and the sensor base assembly is lowered for releasing the sensor base assembly from the ejector assembly.

12. The implantation device according to claim 11, wherein the fifth stopper comprises a fifth sloped surface which is sloped outwardly as extending toward the first end and configured to match with, and abut against, the fourth sloped surface.

13. The implantation device according to claim 11, wherein the ejector assembly comprises a fourth potential energy storage element configured to provide potential energy to the fifth stopper as the ejector assembly moves toward the second end so that the fifth stopper is caused to gradually move outwardly along the radial direction of the base body.

14. The implantation device according to claim 1, wherein the ejector assembly further comprises a fifth potential energy storage element,
when the ejector assembly is assembled with the sensor base assembly, the fifth potential energy storage element stores potential energy,
when the ejector assembly is separated from the sensor base assembly, the fifth potential energy storage element releases potential energy to cause the sensor base assembly to move away from the ejector assembly.

15. A needle assembly, for use with the implantation device according to any one of claims 1 to 14, wherein the needle assembly comprises a shell and a needle body,
the shell connected to the needle body,
the shell configured to be connected to the ejector assembly of the implantation device, the shell also configured to be detachably connected to the sensor base assembly.

16. The needle assembly according to claim 15, further comprising a needle sheath and a sixth potential energy storage element, the sixth potential energy storage element fixedly connected at opposite ends thereof to the needle sheath and the needle body respectively,
when the shell is assembled with the sensor base assembly, the sixth potential energy storage element stores potential energy so that the needle body protrudes out of the needle sheath along an axis of the shell,
when the shell is separated from the sensor base assembly, the sixth potential energy storage element releases potential energy so that the needle body is gradually received into the needle sheath along the axis of the shell.

17. The needle assembly according to claim 16, wherein the needle body is connected to the shell so as to be movable along the axis thereof, and the needle sheath is fixedly connected to the shell,
when the shell is assembled with the sensor base assembly, the sixth potential energy storage element stores potential energy so that the needle body protrudes out of the needle sheath along the axis of the shell, and
when the shell is separated from the sensor base assembly, the sixth potential energy storage element releases potential energy so that the needle body is received into the needle sheath.

18. The needle assembly according to claim 17, wherein the needle body comprises a needle holder, wherein one end of the sixth potential energy storage element is connected to the needle holder, and a further end of the sixth potential energy storage element is connected to the needle sheath.

19. The needle assembly according to claim 16, wherein the needle body is fixedly connected to the shell, and the needle sheath is connected to the shell so as to be movable along the axis of the shell,
when the shell is assembled with the sensor base assembly, the sixth potential energy storage element stores potential energy so that the needle body protrudes out of the needle sheath as the needle sheath is restricted in position by the shell, and
when the shell is separated from the sensor base assembly, the needle sheath is released from the shell, and the sixth potential energy storage element releases potential energy so that the needle sheath is caused to move along the axis of the shell over the needle body.

20. The needle assembly according to claim 16, wherein the shell comprises a sixth stopper,
when the shell is assembled with the sensor base assembly, the sixth stopper is configured to abut against the needle body or the needle sheath to cause the sixth potential energy storage element to store potential energy so that the needle body or the needle sheath is restricted in axial position relative to the shell,
when the shell is separated from the sensor base assembly, the sixth stopper moves away from abutment against the needle body or the needle sheath to cause the sixth potential energy storage element to release potential energy so that the needle body or the needle sheath is released from positional restriction.

21. The needle assembly according to claim 16, wherein the shell comprises a seventh potential energy storage element and a first snap engagement member, the first snap engagement member configured to be detachable connected with the sensor base assembly along the axis of the shell, when the first snap engagement member is assembled with the sensor base assembly, the seventh potential energy storage element stores potential energy and provides potential energy to the first snap engagement member, so that the first snap engagement member is caused to move along a radial direction of the shell to abut against the sensor base assembly.

22. The needle assembly according to claim 21, wherein the shell comprises a sixth stopper, when the first snap engagement member is separated from the sensor base assembly, the seventh potential energy storage element releases potential energy to cause the sixth stopper to move so that the needle body or the needle sheath is released from positional restriction.

23. The needle assembly according to claim 15, wherein the shell is detachably connected to the ejector assembly along an axis thereof, wherein the needle assembly further comprises an eighth potential energy storage element,
when the shell is assembled with the ejector assembly, the eighth potential energy storage element stores potential energy,
when the shell is separated from the ejector assembly, the eighth potential energy storage element releases potential energy to cause the shell to move along the axis of the ejector assembly so that the shell is separated from the ejector assembly.

24. The needle assembly according to claim 23, wherein the shell comprises a seventh stopper configured to abut against the third stopper of the ejector assembly so that the shell is restricted from moving toward the second end and does not exceed the third predetermined position relative to the ejector assembly.

25. The needle assembly according to claim 24, wherein the needle assembly is detachably connected to the ejector assembly, when the seventh stopper moves away from abutment against the third stopper, the shell is no longer restricted from movement, so that the needle assembly is separated from the ejector assembly.

26. A sensor base assembly, for use with the implantation device according to any one of claims 1 to 14 and with the needle assembly according to any one of claims 15 to 25, wherein the sensor base assembly comprises a base and an adhesive layer,
the base having a third end and a fourth end, which oppose each other along an axis of the base, the base configured to be detachably connected, at a side where the third end is located, to the shell of the needle assembly,
wherein the adhesive layer is provided at a side of the base where the fourth end is located.

27. The sensor base assembly according to claim 26, wherein when the ejector assembly is located at the first predetermined position, the base is configured for snap engagement with the fifth stopper to restrict the sensor base assembly from moving toward the second end relative to the ejector assembly,
wherein as the ejector assembly moves from the first predetermined position toward the second end, the base is configured to disengage from the fifth stopper to release the sensor base assembly from the fifth stopper.

28. The sensor base assembly according to claim 26, wherein the base comprises a second snap engagement member configured to be detachable connected with the first snap engagement of the needle assembly along an axis of the shell of the needle assembly.

29. The sensor base assembly according to claim 26, wherein the base is further configured to detachably connected, at the third end, to an emitter assembly, wherein the base comprises a third snap engagement member configured for snap engagement with the emitter assembly.

30. The sensor base assembly according to claim 29, further comprising a flexible conductor and a sensor component, the sensor component configured to be implanted together with the needle body of the needle assembly to a predetermined site so that the flexible conductor is electrically connected to the sensor component and to be detachably connected to the emitter assembly.

31. An emitter assembly, for matching with and connecting to the sensor base assembly according to claim 29 or 30, wherein the emitter assembly comprises a fourth snap engagement member configured for snap engagement with the third snap engagement member of the sensor base assembly.

32. A sterilization box assembly, for accommodating the needle assembly according to any one of claims 15 to 25 and the sensor base assembly according to any one of claims 26 to 30.

33. The sterilization box assembly according to claim 32, comprising a needle recovery member configured to be brought into abutment against the third stopper of the implantation device to release the needle assembly from the third stopper so that the needle assembly is separated from the implantation device.

34. The sterilization box assembly according to claim 33, wherein the sterilization box assembly is further configured to accommodate the needle assembly that has been separated from the implantation device.

35. The sterilization box assembly according to claim 33, comprising a sterilization box shell comprising a first cavity and a second cavity, the first cavity configured for accommodating therein the needle assembly and the sensor base assembly, which are assembled together, wherein the needle recovery member is disposed at the side of the sterilization box shell opposite to the first cavity, and after the needle recovery member is brought into abutment against the third stopper of the implantation device and the needle assembly is released from the third stopper, the needle assembly moves from the implantation device into the second cavity.

36. The sterilization box assembly according to claim 35, further comprising a foil seal configured to be sealingly connected to the sterilization box shell to close the first cavity.

37. The sterilization box assembly according to claim 36, further comprising a top protective cover provided on an inner surface of the foil seal, the top protective cover configured to restrict movement of the needle assembly and the sensor base assembly.

38. The sterilization box assembly according to claim 33, further comprising a retaining receptacle arranged at a bottom of the sterilization box assembly in alignment with the needle assembly that is falling.

39. An implantation system, comprising the implantation device according to any one of claims 1 to 14, the needle assembly according to any one of claims 15 to 25, the sensor base assembly according to any one of claims 26 to 30 and the sterilization box assembly according to any one of claims 32 to 38, wherein:
the sterilization box assembly is configured to accommodate the needle assembly and the sensor base assembly, which are assembled together;
the implantation device is configured to be loaded, along a direction from the second end to the first end, into the needle assembly and the sensor base assembly that are accommodated in the sterilization box assembly;
the needle assembly is configured to be separated from the sensor base assembly after the sensor base assembly is attached to a predetermined implantation site; and
the emitter assembly is configured to be assembled with the sensor base assembly after the needle assembly is separated from the sensor base assembly.
